# EUROPEAN PATENT SPECIFICATION

(11) **EP 3 347 452 B1**
(45) Date of publication and mention of the grant of the patent: **23.06.2021**
(21) Application number: 16769882.8
(22) Date of filing: 06.09.2016
(51) Int. Cl.: A61P 37/00, A61P 37/06, A61P 37/08, C12N 5/0783

(54) **A NEW SUBPOPULATION OF CD8+CD45RCLOW TREGS AND USES THEREOF**
NEUE SUBPOPULATION VON CD8+CD45RCLOW-TREGS UND VERWENDUNGEN DAVON
NOUVELLE SOUS-POPULATION DE LYMPHOCYTES T RÉGULATEURS CD8+CD45RCLOW ET LEURS UTILISATIONS

(30) Priority: 07.09.2015 EP 15306366
(43) Date of publication of application: 18.07.2018
(73) Proprietor: INSERM (Institut National de la Santé et de la Recherche Médicale), 75013 Paris (FR); Universite de Nantes, 44000 Nantes (FR); Centre Hospitalier Universitaire De Nantes, 44000 Nantes (FR)
(72) Inventor: GUILLONNEAU, Carole, 44093 Nantes (FR); ANEGON, Ignacio, 44093 Nantes (FR); BEZIE, Séverine, 44093 Nantes (FR)
(74) Representative: Icosa
(86) International application number: PCT/EP2016/070991
(87) International publication number: WO 2017/042170

(56) References cited:
- WO-A1-2016/009041
- WO-A2-2015/150492
- X. L. LI ET AL: "Mechanism and Localization of CD8 Regulatory T Cells in a Heart Transplant Model of Tolerance", THE JOURNAL OF IMMUNOLOGY, vol. 185, no. 2, 15 July 2010 (2010-07-15) , pages 823-833, XP055249825, US ISSN: 0022-1767, DOI: 10.4049/jimmunol.1000120 cited in the application
- Carole Guillonneau ET AL: "CD40Ig treatment results in allograft acceptance mediated by CD8+CD45RClow T cells, IFN-[gamma], and indoleamine 2,3-dioxygenase", The Journal of Clinical Investigation, 1 April 2007 (2007-04-01), pages 1096-1106, XP055250192, DOI: 10.1172/JCI28801 Retrieved from the Internet: URL:http://www.ncbi.nlm.nih.gov/pmc/articl es/PMC1839240/pdf/JCI0728801.pdf [retrieved on 2016-02-15] cited in the application
- SÉVERINE BÉZIE ET AL: "IL-34 is a Treg-specific cytokine and mediates transplant tolerance", JOURNAL OF CLINICAL INVESTIGATION, vol. 125, no. 10, 1 October 2015 (2015-10-01), pages 3952-3964, XP055249807, US ISSN: 0021-9738, DOI: 10.1172/JCI81227

### SEQUENCE LISTING

<110> INSERM (Institut National de la SantÃ® et de la Recherche MÃ©dicale)
<120> A NEW SUBPOPULATION OF CD8+CD45RClow TREGS AND USES THEREOF
<130> GUILLONN14146AS
<160> 18
<170> BiSSAP 1.3.6
<210> 1
   <211> 15
   <212> PRT
   <213> Artificial Sequence
<220>
   <223> Peptide derived from a MHC class II molecule
<400> 1
   Arg Glu Glu Tyr Ala Arg Phe Asp Ser Asp Val Gly Glu Tyr Arg
   1               5                   10                  15
<210> 2
   <211> 16
   <212> PRT
   <213> Artificial Sequence
<220>
   <223> Peptide derived from a MHC class II molecule
<400> 2
   Asn Arg Glu Glu Tyr Ala Arg Phe Asp Ser Asp Val Gly Glu Tyr Arg
   1               5                   10                  15
<210> 3
   <211> 15
   <212> PRT
   <213> Artificial Sequence
<220>
   <223> Peptide derived from a MHC class II molecule
<400> 3
   Arg Glu Glu Tyr Ala Arg Phe Asp Ser Asp Val Gly Glu Phe Arg
   1               5                   10                  15
<210> 4
   <211> 15
   <212> PRT
   <213> Artificial Sequence
<220>
   <223> Peptide derived from a MHC class II molecule
<400> 4
   Arg Glu Glu Tyr Val Arg Phe Asp Ser Asp Val Gly Glu Tyr Arg
   1               5                   10                  15
<210> 5
   <211> 15
   <212> PRT
   <213> Artificial Sequence
<220>
   <223> Peptide derived from a MHC class II molecule
<400> 5
   Gln Glu Glu Tyr Ala Arg Phe Asp Ser Asp Val Gly Glu Tyr Arg
   1               5                   10                  15
<210> 6
   <211> 15
   <212> PRT
   <213> Artificial Sequence
<220>
   <223> Peptide derived from a MHC class II molecule
<400> 6
   Arg Glu Glu Tyr Ala Arg Phe Asp Ser Asp Val Gly Val Tyr Arg
   1               5                   10                  15
<210> 7
   <211> 16
   <212> PRT
   <213> Artificial Sequence
<220>
   <223> Peptide derived from a MHC class II molecule
<400> 7
   Asn Arg Glu Glu Tyr Ala Arg Phe Asp Ser Asp Val Gly Glu Phe Arg
   1 5 10 15
<210> 8
   <211> 16
   <212> PRT
   <213> Artificial Sequence
<220>
   <223> Peptide derived from a MHC class II molecule
<400> 8
   Asn Arg Glu Glu Tyr Val Arg Phe Asp Ser Asp Val Gly Glu Tyr Arg
   1 5 10 15
<210> 9
   <211> 16
   <212> PRT
   <213> Artificial Sequence
<220>
   <223> Peptide derived from a MHC class II molecule
<400> 9
   Asn Gln Glu Glu Tyr Ala Arg Phe Asp Ser Asp Val Gly Glu Tyr Arg
   1 5 10 15
<210> 10
   <211> 16
   <212> PRT
   <213> Artificial Sequence
<220>
   <223> Peptide derived from a MHC class II molecule
<400> 10
   Asn Arg Glu Glu Tyr Ala Arg Phe Asp Ser Asp Val Gly Val Tyr Arg
   1 5 10 15
<210> 11
   <211> 40
   <212> PRT
   <213> Artificial Sequence
<220>
   <223> Peptide derived from a MHC class II molecule
<400> 11
   Arg Leu Leu Ala Arg Leu Ile Tyr Asn Arg Glu Glu Tyr Ala Arg Phe
   1 5 10 15
   Asp Ser Asp Val Gly Glu Tyr Arg Ala Val Thr Glu Leu Gly Arg Pro
   20 25 30
   Ser Ala Glu Tyr Arg Asn Lys Gln
   35 40
<210> 12
   <211> 16
   <212> PRT
   <213> Artificial Sequence
<220>
   <223> Peptide derived from a MHC class II molecule
<400> 12
   Tyr Leu Arg Tyr Asp Ser Asp Val Gly Glu Tyr Arg Ala Val Thr Glu
   1               5                   10                  15
<210> 13
   <211> 16
   <212> PRT
   <213> Artificial Sequence
<220>
   <223> Peptide derived from a MHC class II molecule
<400> 13
   Asp Ser Asp Val Gly Glu Tyr Arg Ala Val Thr Glu Leu Gly Arg Pro
   1               5                   10                  15
<210> 14
   <211> 12
   <212> PRT
   <213> Artificial Sequence
<220>
   <223> Peptide derived from a MHC class II molecule
<400> 14
   Tyr Leu Arg Tyr Asp Ser Asp Val Gly Glu Tyr Arg
   1               5                   10
<210> 15
   <211> 14
   <212> PRT
   <213> Artificial Sequence
<220>
   <223> Peptide derived from a MHC class II molecule
<400> 15
   Tyr Leu Arg Tyr Asp Ser Asp Val Gly Glu Tyr Arg Ala Val
   1               5                   10
<210> 16
   <211> 14
   <212> PRT
   <213> Artificial Sequence
<220>
   <223> Peptide derived from a MHC class II molecule
<400> 16
   Ser Asp Val Gly Glu Tyr Arg Ala Val Thr Glu Leu Gly Arg
   1               5                   10
<210> 17
   <211> 15
   <212> PRT
   <213> Artificial Sequence
<220>
   <223> Peptide derived from a MHC class II molecule
<400> 17
   Leu Arg Tyr Asp Ser Asp Val Gly Glu Tyr Arg Ala Val Thr Glu
   1               5                   10                  15
<210> 18
   <211> 7
   <212> PRT
   <213> Artificial Sequence
<220>
   <223> Peptide derived from a MHC class II molecule
<400> 18
   Ser Asp Val Gly Glu Tyr Arg
   1               5

## Description

### FIELD OF THE INVENTION:

The invention relates to the field of cell therapy with the identification of a highly suppressive subpopulation of human CDS⁺CD45RC^{low} Tregs.

### BACKGROUND OF THE INVENTION:

Immunosuppressive regimens remain a significant obstacle to long-term survival of the graft and welfare of transplanted patients, and in the last years, improvement of allograft survival has stagnated, mainly because of secondary effects and non-specific immunosuppression (Feng 2008). The identification in human of regulatory populations with superior suppressive capacity and specificity toward donor antigens is of great interest in human transplantation and in a number of diseases with a regulatory T cells (Treg)/ effector T cells (Teff) dysregulation.

Indeed, regulatory T cells, or "Tregs" which encompass Foxp3⁺ Treg cells and CD45RC^{low} Tregs are fundamental in controlling various immune responses in that Tregs can rapidly suppress the activity of other immune cells. In particular, Tregs are crucial for maintaining tolerance by downregulating undesired immune responses to self and non-self antigens. For instance, Treg defects have been discovered in patients with multiple sclerosis (MS), type I diabetes (TlD), psoriasis, myasthenia gravis (MG) and other autoimmune diseases. Similar links may also exist for atopy and allergic diseases. For all these diseases reports exist pointing to a reduced *in vitro* immune suppression of the patient's Treg cells. This has led to an increasing interest in the possibility of using Tregs in immunotherapy to treat or prevent autoimmune diseases, allergies, anti-therapeutic protein immune responses (i.e. factor VIII) and transplantation-related complications.

Hence, there is a particular need for subpopulations of Tregs cells with highly suppressive properties, which are virtually free from CD4⁺ and CD8⁺ effector T cells, in order to obtain an isolated population of Treg cells. Indeed, the highly suppressive isolated Tregs may then be expanded and pulsed with antigens of interest or genetically modified (e.g. in order to express a given chimeric antibody receptor also referred to as CAR or to express a chimeric T cell receptor (TCR)) or other types of chimeric antigen receptors in order to efficiently induce antigen-specific immune tolerance. Such expanded populations of Treg cells are of particular interest in the fields of chronic inflammation, autoimmunity, allergy, transplantation, treatment with therapeutic protein and gene therapy, to avoid degradation of self or therapeutic molecules/tissues by the immune system.

### SUMMARY OF THE INVENTION:

In a first aspect, the invention relates to an isolated population of IFNγ⁺IL-10⁺IL-34⁺ secreting human CD8⁺CD45RC^{low} T regulatory (Treg) cells.

Also described is a method for detecting and/or isolating IFNγ⁺IL-10⁺IL-34⁺ secreting human CD8⁺CD45RC^{low} Treg cells from a biological sample containing human peripheral blood mononuclear cells (PBMCs) or lymphocytes comprising the following steps of: (a) contacting said population of human PBMCs or lymphocytes with means useful for isolating a population of human CD8⁺CD45RC^{low} Treg cells; (b) contacting the isolated population of human CD8⁺CD45RC^{low} Treg cells obtained at step (a) with means useful for isolating IFNγ⁺IL-10⁺IL-34⁺ secreting human CD8⁺CD45RC^{low} Treg cells.

In a further aspect, the invention relates to a method for expanding IFNγ⁺IL-10⁺IL-34⁺ secreting human CD8⁺CD45RC^{low} Treg cells comprising a step of contacting an isolated population of IFNγ⁺IL-10⁺IL-34⁺ secreting human CD8⁺CD45RC^{low} Treg cells with a culture medium suitable for expanding Treg cells. Also described is a method for producing a population of IFNγ+IL,-10+IL-34+ secreting human CD8+CD45RClow Treg cells comprising (i) isolating CD8⁺CD45RC^{low} Treg cells from a biological sample containing human peripheral blood mononuclear cells (PBMCs) or lymphocytes, optionally isolating CD8⁺CD45RC^{low}GITR⁺ Treg cells from a biological sample containing human peripheral blood mononuclear cells (PBMCs) or lymphocytes, (ii) optionally freezing and subsequently thawing the isolated Treg cells, and (iii) expanding said cells in culture.

Further described is an isolated population of expanded IFNγ⁺IL-10⁺IL-34⁺ secreting human CD8⁺CD45RC^{low} Treg cells.

In a further aspect, the invention relates to a pharmaceutical composition comprising an isolated population of IFNγ⁺IL-10⁺IL-34⁺ secreting human CD8⁺CD45RC^{low} Treg cells according to the invention.

In a further aspect, the invention relates to an isolated population of IFNγ⁺IL-10⁺IL-34⁺ secreting human CD8⁺CD45RC^{low} Treg cells according to the invention for use as a medicament.

In a further aspect, the invention relates to an isolated population of IFNγ⁺IL-10⁺IL-34⁺ secreting human CD8⁺CD45RC^{low} Treg cells according to the invention for use in a method for preventing or treating transplant rejection, GVHD (graft versus host disease), chronic inflammatory diseases, autoimmune diseases, unwanted immune response against therapeutic proteins or allergies in a patient in need thereof.

In a last aspect, the invention relates to an *in vitro* method for determining whether a patient is at risk of transplant rejection, GVHD, chronic inflammatory diseases, autoimmune diseases, unwanted immune response against therapeutic proteins or allergies, comprising a step of determining the presence of IFNγ⁺IL-10⁺IL-34⁺ secreting human CD8⁺CD45RC^{low} Treg cells in a biological sample obtained from said patient by a method of the invention, wherein the presence of IFNγ⁺IL-10⁺IL-34⁺ secreting human CD8⁺CD45RC^{low} Treg cells is indicative of a reduced risk of transplant rejection, GVHD, chronic inflammatory diseases, autoimmune diseases, unwanted immune response against therapeutic proteins or allergies.

### DETAILED DESCRIPTION OF THE INVENTION:

The invention results from the identification of highly suppressive subpopulations of CD8⁺ Tregs. In particular, the inventors identified a highly suppressive subpopulation of IFNγ⁺IL-10⁺IL-34⁺secreting subpopulation of CD8⁺CD45RC^{low} Tregs. The inventors demonstrated their superior potential compared to classical CD4⁺CD25^{high}CD127^{low} Tregs on the anti-donor CD4⁺CD25⁻ effector T cell response and the role of IFNγ, IL-10, and IL-34 in their suppressive activity. In addition, the inventors described the specific expansion of IFNγ⁺IL-10⁺IL-34⁺secreting CD8⁺CD45RC^{low} Tregs and their potential as a cell-based therapy in transplantation to inhibit human skin graft rejection and GVHD using humanized mice.

### Treg cells population of the invention

In a first aspect, the invention relates to isolated populations of CD8⁺ Tregs and in particular to an isolated population of IFNγ⁺IL-10⁺IL-34⁺ secreting human CD8⁺CD45RC^{low} T regulatory (Treg) cells. The invention also relates to an isolated population of IFNγ⁺IL-10⁺IL-34⁺ secreting human CD8⁺CD45RC^{low} T regulatory (Treg) cells, wherein said Treg cells are GITR⁺ and/or Foxp3⁺.

Also described is an isolated population of CD8+CD45RClow Tregs, an isolated population of CD8+GITR+ Tregs, an isolated population of CD8+Foxp3+ Tregs, an isolated population of CD8+CD45RClowGITR+ Tregs, an isolated population of CD8+CD45RClowFoxp3+ Tregs, an isolated population of CD8⁺GITR⁺Foxp3⁺ Tregs and an isolated population of CD8⁺CD45RC^{low}GITR⁺Foxp3⁺Tregs.

The isolated populations of CD8⁺ Tregs may secrete at least one cytokine among IFNγ, IL-10, and IL-34. Thus, the isolated populations of CD8⁺ Tregs may secrete IFNγ, or IL-10, or IL-34.

The isolated populations of CD8⁺ Tregs may also secrete at least two cytokines among IFNγ, IL-10, and IL-34. Thus, the isolated populations of CD8⁺ Tregs may secrete IFNγ and IL-10, or IFNγ and IL-34, or IL-10 and IL-34.

As mentioned above, the isolated populations of CD8⁺ Tregs according to the invention secrete IFNγ, IL-10, and IL-34.

The isolated populations of CD8⁺ Tregs described hereinabove may further secrete IL-2 and/or TGFβ-1.

In particular, among the human CD8⁺CD45RC^{low} T regulatory cells, the inventors identified a subpopulation of CD8⁺CD45RC^{low} T regulatory cells secreting IFNγ, and IL-10, and IL-34. Thus, in one embodiment, the human CD8⁺CD45RC^{low} Tregs of the invention secrete IFNγ, IL-10, and IL-34.

In one embodiment, the human CD8⁺CD45RC^{low} Tregs of the invention secrete IFNγ, IL-10, and IL-34 and express GITR. In one embodiment, the human CD8⁺CD45RC^{low} Tregs of the invention secrete IFNγ, IL-10, and IL-34 and express Foxp3. In one embodiment, the human CD8⁺CD45RC^{low} Tregs of the invention secrete IFNγ, IL-10, IL-34 and TGFβ-1.

In another embodiment, the human CD8⁺CD45RC^{low} Tregs of the invention secrete IFNγ, IL-10, and IL-34 and express Foxp3 and GITR. In one embodiment, the human CD8⁺CD45RC^{low} Tregs of the invention secrete IFNγ, IL-10, IL-34 and TGFβ-1, and express Foxp3. In another embodiment, the human CD8⁺CD45RC^{low} Tregs of the invention secrete IFNγ, IL-10, IL-34 and TGFβ-1, and express GITR.

In yet another embodiment, the human CD8⁺CD45RC^{low} Tregs of the invention secrete IFNγ, IL-10, IL-34 and TGFβ-1, and express Foxp3 and GITR.

In one embodiment, the human IFNγ⁺IL-10⁺IL-34⁺ secreting CD8⁺CD45RC^{low} Tregs as described hereinabove secrete IL-2.

The isolated population of CD8⁺ Tregs as described hereinabove may be frozen to be stored. In a particular embodiment of the invention, the isolated population of IFNγ⁺IL-10⁺IL-34⁺ secreting human CD8⁺CD45RC^{low} Tregs as described hereinabove may be frozen to be stored. The isolated population of human CD8⁺ Tregs as described hereinabove may have been frozen and thawed. In a particular embodiment, the isolated population of IFNγ⁺IL-10⁺IL-34⁺ secreting human CD8⁺CD45RC^{low} Tregs as described hereinabove has been frozen and thawed.

As used herein, the term "regulatory T cells" or "Tregs", formerly known as suppressor T cells, refers to a subpopulation of T cells which modulate the immune system, maintain tolerance to self-antigens, and abrogate autoimmune diseases. These cells generally suppress or downregulate induction and proliferation of effector T cells.

As used herein, the term "population" refers to a population of cells, wherein the majority (e.g., at least about 50%, preferably at least about 60%, more preferably at least about 70%, and even more preferably at least about 80%) of the total number of cells have the specified characteristics of the cells of interest and express the markers of interest (e.g. a population of human CD8⁺CD45RC^{low} Treg cells comprises at least about 50%, preferably at least about 60%, more preferably at least about 70%, and even more preferably at least about 80% of cells which have the highly suppressive functions and which express the particular markers of interest, such as IFNγ, IL-10, IL-34, Foxp3, GITR or TGFβ-1⁺). As used herein, the term "about" preceding a figure means plus or minus 10% of the value of said figure.

As used herein, "isolated" refers to a cell or a cell population that is removed from its natural environment (such as the peripheral blood) and that is isolated, purified or separated, and is at least about 75% free, 80% free, 85% free and preferably about 90%, 95%, 96%, 97%, 98%, 99% free, from other cells with which it is naturally present, but which lack the cell surface markers based on which the cells were isolated.

As used herein, the term "marker" refers to a protein, glycoprotein, or any other molecule expressed on the surface of a cell or into a cell or secreted by a cell, and which can be used to help identify the cell. A marker can generally be detected by conventional methods. Specific, non-limiting examples of methods that can be used for the detection of a cell surface marker are immunocytochemistry, fluorescence activated cell sorting (FACS), and enzymatic analysis but also RT-PCR and molecular biology methods to detect mRNA of the protein.

As used herein, the term "CD8" (cluster of differentiation 8) well known in the art refers to a transmembrane glycoprotein that serves as a co-receptor for the T cell receptor (TCR). To function, CD8 forms a dimer, consisting of a pair of CD8 chains. The most common form of CD8 is composed of a CD8-α and CD8-β chain. The naturally occurring human CD8-α protein has an amino acid sequence provided in the UniProt database under accession number P01732. The naturally occurring human CD8-β protein has an amino acid sequence provided in the UniProt database under accession number P10966.

As used herein, the term "CD45" (also known as LCA or PTPRC) refers to a transmembrane glycoprotein existing in different isoforms previously described in Streuli *et al.,* 1996. These distinct isoforms of CD45 differ in their extracellular domain structures which arise from alternative splicing of 3 variable exons coding for part of the CD45 extracellular region. The various isoforms of CD45 have different extracellular domains, but have the same transmembrane and cytoplasmic segments having two homologous, highly conserved phosphatase domains of approximately 300 amino acid residues. The naturally occurring human CD45 protein has an amino acid sequence provided in the UniProt database under accession number P08575. As used herein, the term "CD45RC" refers to the exon 6 splice variant (exon C) of the tyrosine phosphatase CD45. The CD45RC isoform is expressed on B cells, and on subsets of CD4⁺ and CD8⁺ T cells.

As used herein, the terms "Interferon gamma" or "IFNγ", or "IFNg", or "type II interferon" are well known in the art and refer to a cytokine that is critical for innate and adaptive immunity. The naturally occurring human IFNγ protein has an amino acid sequence of 146 amino acids provided in the UniProt database under accession number P01579.

According to the invention, the IFNγ secreting CD8⁺ Tregs of the invention secrete at least about 1000, 2000, 3000, 4000, 5000, 6000, 7000, 8000, 9000, 10000, 11000, 12000, 13000, 14000, 15000, 16000, 17000, 18000, 19000 or 20000 pg/ml IFNγ.

As used herein, the terms "Interleukin-10" or "IL-10" are well known in the art and refer to an anti-inflammatory cytokine. The naturally occurring human IL-10 protein has an amino acid sequence of 178 amino acids provided in the UniProt database under accession number P22301.

According to the invention, the IL-10 secreting CD8⁺ Tregs of the invention secrete at least about 0.1, 0.5, 1, 10, 25, 50, 75, 100, 125, 150, 175, 200, 250, 300, 350, 400, 450, 500, 550, 600, 650, 700, 750, 800, 850, 900, 950, 1000, 1100, 1200, 1300, 1400, 1500, 1600, 1700, 1800, 1900 or 2000 pg/ml IL-10.

As used herein, the terms "Interleukin-34" or "IL-34" are well known in the art and refer to a cytokine that promotes the proliferation, survival and differentiation of monocytes and macrophages. The naturally occurring human IL-34 protein has an amino acid sequence of 242 amino acids provided in the UniProt database under accession number Q6ZMJ4.

According to the invention, the IL-34 secreting CD8⁺ Tregs of the invention secrete at least about 0.1, 0.5, 1, 10, 25, 50, 75, 100, 125, 150, 175, 200, 250, 300, 350, 400, 450, 500, 550, 600, 650, 700, 750, 800, 850, 900, 950, 1000, 1100, 1200, 1300, 1400, 1500, 1600, 1700, 1800, 1900 or 2000 pg/ml IL-34.

As used herein, the terms "glucocorticoid-induced TNFR-related protein" or "GITR", or "Tumor necrosis factor receptor superfamily member 18", or "TNFRSF18" are well known in the art and refer to a surface receptor molecule of the TNF receptor superfamily. The naturally occurring human GITR has an amino acid sequence of 241 amino acids provided in the UniProt database under accession number Q9Y5U5.

As used herein, the terms "forkhead box P3" or "Foxp3" are well known in the art and refer to a nuclear protein believed to act as a transcription factor, marker of T regulatory cells. The naturally occurring human Foxp3 has an amino acid sequence of 431 amino acids provided in the UniProt database under accession number Q9BZS1.

As used herein, the terms "Transforming growth factor beta 1" or "TGFβ-1", or "TGFb1", are well known in the art and refer to a cytokine that plays an important role in controlling the immune system. The naturally occurring human TGFβ-1 has an amino acid sequence of 390 amino acids provided in the UniProt database under accession number P01137.

According to the invention, the TGFβ-1 secreting CD8⁺ Tregs of the invention secrete at least about 0.1, 0.5, 1, 10, 25, 50, 75, 100, 125, 150, 175, 200, 250, 300, 350, 400, 450, 500, 550, 600, 650, 700, 750, 800, 850, 900, 950, 1000, 1100, 1200, 1300, 1400, 1500, 1600, 1700, 1800, 1900 or 2000 pg/ml TGFβ-1.

As used herein, the terms "Interleukin-2" or "IL-2", are well known in the art and refer to a cytokine that plays crucial roles in regulating both immune activation and homeostasis. The naturally occurring human IL-2 has an amino acid sequence of 153 amino acids provided in the UniProt database under accession number P60568.

According to the invention, the IL-2 secreting CD8⁺ Tregs as described hereinabove secrete at least about 0.1, 0.5, 1, 10, 25, 50, 75, 100, 125, 150, 175, 200, 250, 300, 350, 400, 450, 500, 550, 600, 650, 700, 750, 800, 850, 900, 950, 1000, 1100, 1200, 1300, 1400, 1500, 1600, 1700, 1800, 1900, or 2000 pg/ml IL-2.

The cytokine secretion may typically be evaluated in cultures of cells after activation with at least one stimulating agent. Examples of stimulating agents include, but are not limited to, antigens, cells, MHC polymers, antibodies and lectins. Said at least one stimulating agent may be selected from the group comprising allogeneic antigen presenting cells, anti-CD3 antibodies and/or anti-CD8 antibodies, IL-2, PMA + ionomycine.

The cytokine secretion may typically be evaluated after 6, 7, 8, 9, 10, 11, 12, 13, 14, 15, 16, 17, 18, 19, 20, or 21 days of culture, preferably after 12, 13, 14, 15, 16, or 17 days of culture and more preferably after 14 days of culture. Furthermore, the cytokine secretion may typically be evaluated in a culture started with about 2 x 10⁵, 3 x 10⁵, or 4 x 10⁵ cellules/ml, preferably with about 2.5 x 10⁵, 3 x 10⁵, or 3.5 x 10⁵ cellules/ml, more preferably with about 3 x 10⁵ cellules/ml. In particular, the cytokine secretion may be evaluated after 14 days of culture in a culture started with about 3.5 x 10⁵ cellules/ml.

In one embodiment of the invention, the IFNγ⁺IL-10⁺IL-34⁺ secreting human CD8⁺CD45RC^{low} T regulatory (Treg) cells as described hereinabove, may be genetically modified to encode desired expression products, as will be further described below.

The term "genetically modified" indicates that the cells comprise a nucleic acid molecule not naturally present in non-modified IFNγ⁺IL-10⁺IL-34⁺ secreting human CD8⁺CD45RC^{low} Treg cells, or a nucleic acid molecule present in a non-natural state in said CD8⁺ human Tregs of the invention, in particular in IFNγ⁺IL-10⁺IL-34⁺ secreting human CD8⁺CD45RC^{low} Treg cells (e.g., amplified). The nucleic acid molecule may have been introduced into said cells or into an ancestor thereof.

A number of approaches can be used to genetically modify he IFNγ⁺IL-10⁺IL-34⁺ secreting human CD8⁺CD45RC^{low} Treg cells, such as virus-mediated gene delivery, non-virus-mediated gene delivery, naked DNA, physical treatments, etc. To this end, the nucleic acid is usually incorporated into a vector, such as a recombinant virus, a plasmid, phage, episome, artificial chromosome, etc. Examples of means by which the nucleic acid carrying the gene may be introduced into the cells include, but are not limited to, microinjection, electroporation, transduction, or transfection using DEAE-dextran, lipofection, calcium phosphate or other procedures known to one skilled in the art.

In a particular embodiment of the invention, the IFNγ⁺IL-10⁺IL-34⁺ secreting human CD8⁺CD45RC^{low} Treg cells are genetically modified using a vector particle such as a viral vector (or a recombinant virus) or a virus-like particle (VLP). In this embodiment, the heterologous nucleic acid is, for example, introduced into a recombinant virus which is then used to infect the IFNγ⁺IL-10⁺IL-34⁺ secreting human CD8⁺CD45RC^{low} Treg cells. Different types of recombinant viruses can be used, in particular recombinant retroviruses. Retroviruses are preferred vectors since retroviral infection results in stable integration into the genome of the cells. This is an important property because lymphocyte expansion, either *in vitro* or *in vivo* after injection into the subject, requires that the transgene is maintained stable during segregation in order to be transmitted at each cell division. Examples of retrovirus types which can be used are retroviruses from the oncovirus, lentivirus or spumavirus family. Particular examples of the oncovirus family are slow oncovirus, non oncogene carriers, such as MoMLV, ALV, BLV or MMTV, and fast oncoviruses, such as RSV. Examples from the lentivirus family are HIV, SIV, FIV or CAEV.

Techniques for constructing defective recombinant retroviruses have been widely described in the literature (WO 89/07150, WO 90/02806, and WO 94/19478). These techniques usually comprise the introduction of a retroviral vector comprising the transgene into an appropriate packaging cell line, followed by the recovery of the viruses produced, said viruses comprising the transgene in their genome. The IFNγ⁺IL-10⁺IL-34⁺ secreting human CD8⁺CD45RC^{low} Treg cells can be infected with recombinant viruses using various protocols, such as by incubation with a virus supernatant, with purified viruses, by co-culturing said Treg cells with the virus' packaging cells, by Transwell techniques, etc.

As used herein, the term "Virus-Like Particle" (VLP) refers to a structure resembling a virus particle. A virus-like particle in accordance with the invention is non-replicative since it lacks all or part of the viral genome, typically and preferably lacks all or part of the replicative and infectious components of the viral genome. The term "non-replicative", as used herein, refers to being incapable of replicating the genome comprised or not in the VLP. VLP may be prepared according to techniques known in the art and for example as described in the international patent application published under n° WO 02/34893.

The nucleic acid used to genetically modify the IFNγ⁺IL-10⁺IL-34⁺ secreting human CD8⁺CD45RC^{low} Treg cells may encode various biologically active products, including polypeptides (e.g., proteins, peptides, etc.), RNAs, etc. In a particular embodiment, the nucleic acid encodes a polypeptide having an immuno-suppressive activity. In another embodiment, the nucleic acid encodes a polypeptide which is toxic or conditionally toxic to the cells. Preferred examples include a thymidine kinase (which confers toxicity in the presence of nucleoside analogs), such as HSV-1 TK, a cytosine desaminase, etc.

Another preferred category of nucleic acids are those encoding a T cell receptor or a subunit or functional equivalent thereof such as a chimeric antigen receptor (CAR) specific to an antigen of interest or a chimeric autoantibody receptor (CAAR) comprising an auto-antigen. For instance, the expression of recombinant TCRs or CARs specific for an antigen produces IFNγ⁺IL-10⁺IL-34⁺ secreting human CD8⁺CD45RC^{low} Treg cells which can act more specifically and efficiently on effector T cells to inhibit immune responses in a patient in need thereof. The basic principles of chimeric antigen receptor (CAR) design have been extensively described (e.g. Sadelain *et al.,* 2013).

CARs comprise an extracellular antigen-recognition moiety generally linked via spacer/hinge and a transmembrane domain to an intracellular signaling domain. The intracellular signaling domain of "first generation" CARs only comprise a T-cell activation moiety. The intracellular domain of "second generation" CARs comprise a co-stimulatory moiety in tandem with an activation moiety, for example CD3ζ. Examples of co-stimulatory domains include, but are not limited to, ICOS, OX40 (CD134), CD28, 4-1BB (CD137), CD27 and DAP10. The intracellular domain of "third generation" CARs comprise two co-stimulatory domains in tandem with an activation moiety, such as the combination of CD28, a tumor necrosis factor receptor (TNFr), such as OX40 or 4-1BB, and CD3 ζ.

CARs are generally obtained by fusing the extracellular antigen-binding domain with the intracellular signaling domains derived from the CD3-ζ chain of the T-cell receptor, in tandem with costimulatory endo-domains to support survival and proliferative signals. Because CAR-modified T cells function independently of a patient's MHC and can readily be generated for clinical use, the targeting of pathogenic antigens as described below with a CAR based-approach is useful.

CAARs comprise an extracellular autoantigen, such as an autoantigen involved in an autoimmune disease, fused to intracellular signaling domains. Examples of the intracellular signaling domains of a CAAR include, without being limited to, T or NK receptor signaling domains such as CD137CD3ζ signaling domain.

In one embodiment, the CD8+ Tregs of the invention are genetically modified and express at least one CAR, one CAAR and/or one native receptor linked to intracellular signaling molecules. Examples of CAR included, without being limited to, first generation CARs, second generation CARs, third generation CARs, CARs comprising more than three signaling domains (co-stimulatory domains and activation domain), and inhibitory CARs (iCARs).

Thus, in one embodiment, the invention relates to an isolated population of IFNγ⁺IL-10⁺IL-34⁺ secreting human CD8⁺CD45RC^{low} Treg cells, wherein said Treg cells are genetically modified Treg cells comprising a chimeric antigen receptor.

According to the present invention, the extra-cellular domain of the CAR recognizing an antigen of interest may comprise a receptor, or a fragment of a receptor, which binds to said antigen, such as an antibody or an antigen-binding fragment thereof. According to the present invention, the extra-cellular domain of the CAR may comprise a human antibody or an antibody originating from any other species.

As used herein, the term "antibody fragment" refers to at least one portion of an antibody that retains the ability to specifically interact with an epitope of an antigen. Examples of antibody fragments include, without being limited to, Fab, Fab', F(ab')2, Fv fragments, scFv antibody fragments, disulfide-linked Fvs (sdFv), a Fd fragment consisting of the VH and CHI domains, linear antibodies, single domain antibodies such as sdAb (either VL or VH), camelid VHH domains, multi-specific antibodies formed from antibody fragments such as a bivalent fragment comprising two Fab fragments linked by a disulfide bridge at the hinge region, and an isolated CDR or other epitope binding fragments of an antibody.

In another embodiment, the CD8+ Tregs of the invention are genetically modified and lack expression of a functional T cell receptor (TCR) and/or human leukocyte antigen (HLA), e.g., HLA class I and/or HLA class II. In one embodiment, the genetically modified CD8+ Tregs of the invention lacking a functional TCR and/or HLA are allogeneic Tregs.

### Means for capturing the Treg cells of the invention:

The subpopulation of CD8⁺CD45RC^{low} Treg cells of the invention as described hereinabove may be operationally characterized by their cell surface markers and their capacity to produce IFNγ, IL-10 and IL-34.

Thus also described is a method for detecting and/or isolating the subpopulation of IFNγ⁺IL-10⁺IL-34⁺ secreting human CD8⁺CD45RC^{low} Treg cells as described hereinabove from a biological sample containing human peripheral blood mononuclear cells (PBMCs) or lymphocytes comprising the following steps of: (a) contacting said population of human PBMCs or lymphocytes with means useful for isolating a population of human CD8⁺CD45RC^{low} Treg cells; (b) contacting the isolated population of human CD8⁺CD45RC^{low} Treg cells obtained at step (a) with means useful for isolating IFNγ⁺IL-10⁺IL-34⁺ secreting human CD8⁺CD45RC^{low} Treg cells.

Further described is method for detecting and/or isolating IFNγ⁺IL-10⁺IL-34⁺ secreting human CD8⁺CD45RC^{low} Treg cells from a biological sample containing human peripheral blood mononuclear cells (PBMCs) or lymphocytes, wherein said Treg cells optionally are GITR⁺ and/or Foxp3⁺, comprising the following steps of: (a) contacting said population of human PBMCs or lymphocytes with means useful for isolating a population of human CD8⁺CD45RC^{low} Treg cells, optionally with means useful for isolating a population of GITR⁺ and/or Foxp3⁺ CD8⁺CD45RC^{low} Treg cells; (b) contacting the isolated population of human CD8⁺CD45RC^{low} Treg cells obtained at step (a) with means useful for isolating IFNγ⁺IL-10⁺IL-34⁺ secreting human CD8⁺CD45RC^{low} Treg cells.

Step (b) of the method may comprise contacting the isolated population of human CD8⁺CD45RC^{low} Treg cells obtained at step (a) with means useful for isolating IFNγ⁺IL-10⁺IL-34⁺ secreting human CD8⁺CD45RC^{low} Treg cells that also express at least one of GITR, Foxp3 and TGFβ-1.

Alternatively, step (b) of the method may comprise contacting the isolated population of human CD8⁺CD45RC^{low} Treg cells obtained at step (a) with means useful for isolating IFNγ⁺IL-10⁺IL-34⁺ secreting human CD8⁺CD45RC^{low} Treg cells that also express Foxp3 and GITR, or Foxp3 and TGFβ-1, or GITR and TGFβ-1.

Step (b) of the method may also comprise contacting the isolated population of human CD8⁺CD45RC^{low} Treg cells obtained at step (a) with means useful for isolating IFNγ⁺IL-10⁺IL-34⁺ secreting human CD8⁺CD45RC^{low} Treg cells that also express GITR and Foxp3 and TGFβ-1.

The isolation of a population of human CD8⁺CD45RC^{low} Treg cells at step (a) of the method may not require the activation of the CD8⁺ Treg cells of the invention. According to one embodiment, the isolation of IFNγ⁺IL-10⁺IL-34⁺ secreting human CD8⁺CD45RC^{low} Treg cells of the invention at step (b) of the method is carried out after stimulation of the isolated population of human CD8⁺CD45RC^{low} Treg cells obtained at step (a) with at least one stimulating agent. Examples of stimulating agents are described hereinabove.

As used herein, the term "biological sample" refers to any body fluid or tissue that contains peripheral blood mononuclear cells "PBMCs" or lymphocytes. Within the context of the invention, a "PBMC or lymphocyte containing fluid" encompasses blood (whole blood sample, serum sample, or plasma sample) and synovial fluid. A "lymphocyte containing tissue" encompasses spleen, thymus, lymph nodes, bone marrow, Peyer's patches, and tonsils.

The method may be for obtaining and/or isolating IFNγ⁺IL-10⁺IL-34⁺ secreting human CD8⁺CD45RC^{low} Treg cells as described hereinabove from a culture of induced pluripotent stem cells (iPSC) or iPSC derived CD34⁺ cells. A culture of iPSC or iPSC derived CD34⁺ cells may be obtained, for example, from a biological sample containing human peripheral blood mononuclear cells (PBMCs).

At steps (a) and (b), isolation of the population of human cells of interest may be carried out by a variety of methods for detecting a particular immune cell population available for a skilled artisan, including immunoselection techniques, such as high-throughput cell sorting using flow cytometric methods, affinity methods with antibodies labeled to magnetic beads, biodegradable beads, non-biodegradable beads, and combination of such methods.

As used herein, the term "flow cytometric methods" refers to a technique for counting cells of interest, by suspending them in a stream of fluid and passing them through an electronic detection apparatus. Flow cytometric methods allow simultaneous multiparametric analysis of the physical and/or chemical parameters of up to thousands of particles per second, such as fluorescent parameters. Modern flow cytometric instruments usually have multiple lasers and fluorescence detectors. A common variation of flow cytometric techniques is to physically sort particles based on their properties, so as to purify or detect populations of interest, using "fluorescence-activated cell sorting".

As used herein, "fluorescence-activated cell sorting" (FACS) refers to a flow cytometric method for sorting a heterogeneous mixture of cells from a biological sample into two or more containers, one cell at a time, based upon the specific light scattering and fluorescent characteristics of each cell. FACS provides fast, objective and quantitative recording of fluorescent signals from individual cells as well as physical separation of cells of particular interest. Accordingly, FACS can be used with the methods described herein to isolate for instance human CD8⁺ Treg cells.

Alternatively, isolation of human CD8⁺CD45RC^{low} Treg cells according to the invention can be performed using bead based sorting methods, such as magnetic beads.

Using such methods, cells can be separated and isolated positively or negatively with respect to particular cell-surface markers. As defined herein, "positive selection" refers to techniques that result in the isolation and detection of cells expressing specific cell-surface markers, while "negative selection" refers to techniques that result in the isolation and detection of cells not expressing specific cell-surface markers. In some embodiments, beads can be coated with antibodies by a skilled artisan using standard techniques known in the art, such as commercial bead conjugation kits. In some embodiments, a negative selection step is performed to remove cells expressing one or more lineage markers, followed by fluorescence-activated cell sorting to positively select human Treg cells of interest (i.e. IFNγ⁺IL-10⁺IL-34⁺ secreting human CD8⁺CD45RC^{low} Tregs as described hereinabove).

Thus, the method may comprise: (a) contacting a population of human PBMCs or lymphocytes with means useful for depleting non-CD8⁺CD45RC^{low} Treg cells; (b) contacting the isolated population of human CD8⁺CD45RC^{low} Treg cells obtained at step (a) with means useful for isolating IFNγ⁺IL-10⁺IL-34⁺ secreting human CD8⁺CD45RC^{low} Treg cells.

Typically, means useful for isolating a population of cells such as a population of human CD8⁺CD45RC^{low} Treg cells are binding partners (such as antibodies) to suitable cell surface molecules or markers.

Specific binding partners include capture moieties and label moieties. The capture moieties are those which attach both to the cell, either directly or indirectly, and the product. The label moieties are those which attach to the product and may be directly or indirectly labeled. Specific binding partners include any moiety for which there is a relatively high affinity and specificity between product and its binding partner, and for which the dissociation of the product:partner complex is relatively slow so that the product:partner complex is detected during the labeling or cell separation technique.

The capture moiety may be coupled to the anchoring means (the "anchor moiety") optionally through a linking moiety, and may also include a linking moiety which multiplies the number of capture moieties available and thus the potential for capture of product, such as branched polymers, including, for example, modified dextran molecules, polyethylene glycol, polypropylene glycol, polyvinyl alcohol, and polyvinylpyrrolidone.

The label moieties that can be conjugated to a capture moiety such as an antibody are well known to the skilled in the art. For example, radioisotopes, e.g. ³²P, ³⁵S or ³H; fluorescence or luminescence markers, e.g. fluorescein (FITC), rhodamine, texas red, phycoerythrin (PE), allophycocyanin, peridinin-chlorophyll-protein complex (PerCP), 6-carboxyfluorescein (6-FAM), 2', 7'- dimethoxy-4', 5'-dichloro-6-carboxyfluorescein (JOE), 6-carboxy-X-rhodamine (6-ROX), 6-carboxy-2', 4', 7', 4, 7-hexachlorofluorescein (HEX), 5-carboxyfluorescein (5-FAM) or N, N, N', N' -tetramethyl-6-carboxyrhodamine (TAMRA); antibodies or antibody fragments, e.g. F(ab)2 fragment; affinity labels, e.g. biotin, avidin, agarose, bone morphogenetic protein (BMP), matrix bound, haptens; and enzymes or enzyme substrates, e.g. alkaline phosphatase (AP) and horseradish peroxidase (HRP).

Suitable cell molecules include, but are not limited to, cell surface markers of human CD8⁺CD45RC^{low} Treg cells such as CD3 (T cells (activating)), CD8 and CD45RC as well as other CD markers or cell adhesion molecules specific for these CD8⁺ T cells.

In one embodiment of the invention, at step (a) the means useful for isolating a population of human CD8⁺CD45RC^{low} Treg cells are antibodies useful for isolating a population of human CD8⁺CD45RC^{low} Treg cells.

In one embodiment of the invention, at step (a) the means useful for isolating the population of human CD8⁺CD45RC^{low} Treg cells are a combination of at least three antibodies consisting of a monoclonal anti-human CD3 antibody, a monoclonal anti-human CD8 antibody and a monoclonal anti-human CD45RC antibody, optionally further comprising an anti-human GITR antibody (preferably a monoclonal anti-human GITR antibody).

In one embodiment of the invention, at step (a) the means useful for isolating the population of human CD8⁺CD45RC^{low} Treg cells are a combination of at least three antibodies consisting of a monoclonal anti-human CD3 antibody, a monoclonal anti-human CD8 antibody and a monoclonal anti-human CD45RC antibody, optionally further comprising an anti-human GITR antibody (preferably a monoclonal anti-human GITR antibody) and/or an anti-human Foxp3 antibody (preferably a monoclonal anti-human Foxp3 antibody).

As used herein, the term "antibody" refers to immunoglobulin molecules and immunologically active portions of immunoglobulin molecules, i.e., molecules that contain an antigen binding site that immuno-specifically binds an antigen. An "anti-X antibody" or "X antibody" according to the invention is an antibody which can specifically bind to X.

In a particular embodiment of the invention, the antibodies useful for isolating a population of human CD8⁺CD45RC^{low} Treg cells are an anti-human CD3 antibody, an anti-human CD8 antibody and an anti-human CD45RC antibody, preferably a monoclonal anti-human CD3 antibody, a monoclonal anti-human CD8 antibody and a monoclonal anti-human CD45RC antibody. Examples of antibodies which bind the human CD3 antigen that are contemplated by the invention include the monoclonal antibodies OKT3, UCHT1, HIT3a, S4.1 and SK7. Examples of antibodies which bind the human CD8 antigen that are contemplated by the invention include the monoclonal antibodies C8/144B, SKI, 3B5, OKT8, BW135/80 and RPA-T8. Examples of antibodies which bind the human CD45RC antigen that are contemplated by the invention include the monoclonal antibodies MT2 and RP1/12.

In one embodiment of the invention, at step (b) the means useful for isolating a population of IFNγ⁺IL-10⁺IL-34⁺ secreting human CD8⁺CD45RC^{low} Treg cells are antibodies useful for isolating a population of IFNγ⁺IL-10⁺IL-34⁺ secreting human CD8⁺CD45RC^{low} Treg cells.

More particularly, the antibodies useful for isolating a population of human IFNγ⁺IL-10⁺IL-34⁺ secreting human CD8⁺CD45RC^{low} Treg cells are bispecific antibodies which bind to one of the cytokines secreted by the human CD8⁺CD45RC^{low} Treg cells of the invention (i.e. IFNγ, IL-10 and IL-34) and to a cell surface molecule specific for T cells, preferably for CD8⁺ T cells (e.g. CD3, CD8, CD45). Examples of bispecific antibodies that are contemplated by the invention include a bispecific antibody which binds to human IFNγ and human CD45, a bispecific antibody which binds to human IFNγ and human CD3, a bispecific antibody which binds to human IFNγ and human CD8, a bispecific antibody which binds to human IL-10 and human CD45, a bispecific antibody which binds to human IL-10 and human CD3, a bispecific antibody which binds to human IL-10 and human CD8, a bispecific antibody which binds to human IL-34 and human CD45, a bispecific antibody which binds to human IL-34 and human CD3, a bispecific antibody which binds to human IL-34 and human CD8. For instance, IFNγ or IL-10 Secretion assay-detection kits are commercially available (Miltenyi biotec).

In one embodiment, for isolating a population of human IFNγ⁺IL-10⁺IL-34⁺ secreting human CD8⁺CD45RC^{low} Treg cells as described hereinabove, step (b) of the method of the invention comprises a combination of at least two bispecific antibodies selected from the group consisting of a bispecific antibody which binds to IFNγ and to a cell surface molecule specific for T cells, preferably for CD8⁺ T cells (e.g. CD3, CD8, CD45), a bispecific antibody which binds to IL-10 and to a cell surface molecule specific for T cells, preferably for CD8⁺ T cells (e.g. CD3, CD8, CD45) and a bispecific antibody which binds to IL-34 and to a cell surface molecule specific for T cells, preferably for CD8⁺ T cells (e.g. CD3, CD8, CD45).

Amongst IFNγ⁺ secreting human CD8⁺CD45RC^{low} Treg cells, only 10 or 20% are IL-10⁺. According to the results obtained by the Applicant, IL-10 and IL-34 are generally co-expressed by human CD8⁺CD45RC^{low} Treg cells. Therefore, in on embodiment, the combination of bispecific antibodies to isolate the IFNγ⁺IL-10⁺IL-34⁺ secreting human CD8⁺CD45RC^{low} Tregs from the CD8⁺CD45RC^{low} Tregs isolated at step (a) comprises at least a bispecific antibody which binds to IFNγ and to a cell surface molecule specific for T cells and either a bispecific antibody which binds to IL-10 and to a cell surface molecule specific for T cells or a bispecific antibody which binds to IL-34 and to a cell surface molecule specific for T cells.

In a particular embodiment, a bispecific antibody which binds to IFNγ and to a cell surface molecule specific for T cells, a bispecific antibody which binds to IL-10 and to a cell surface molecule specific for T cells and a bispecific antibody which binds to IL-34 and to a cell surface molecule specific for T cells are used in order to isolate the population of interest.

Bispecific antibodies, also known as bifunctional antibodies, have at least one antigen recognition site for a first antigen and at least one antigen recognition site for a second antigen. Such antibodies can be produced by recombinant DNA methods or chemically by methods known in the art. Chemically created bispecific antibodies include, but are not limited to, antibodies that have been reduced and reformed so as to retain their bivalent characteristics and antibodies that have been chemically coupled so that they have at least two antigen recognition sites for each antigen. Bispecific antibodies include all antibodies or conjugates of antibodies, or polymeric forms of antibodies which are capable of recognizing two different antigens. Antibodies can be immobilized on a polymer or particle.

Techniques for obtaining such bispecific antibodies have been extensively described in the international patent application WO 94/09117.

In one embodiment, for isolating a population of human IFNγ⁺IL-10⁺IL-34⁺ secreting human CD8⁺CD45RC^{low} Treg cells as described hereinabove, step (b) of the method of the invention comprises the use of a trispecific antibody which binds to IFNγ, to IL-10 and to a cell surface molecule specific for T cells.

Trispecific antibodies that co-engage three antigens have been described in WO2016105450.

In one embodiment of the invention, at step (b) the means useful for isolating a population of IFNγ⁺IL-10⁺IL-34⁺ secreting human GITR⁺CD8⁺CD45RC^{low} Treg cells are antibodies useful for isolating a population of IFNγ⁺IL-10⁺IL-34⁺ secreting human CD8⁺CD45RC^{low} Treg cells as described hereinabove and antibodies useful for detecting GITR and/or Foxp3 and/or TGFβ-1.

More particularly, the antibodies useful for detecting GITR are human anti-GITR antibodies. Examples of antibodies which bind the human GITR antigen include, without being limited to, the monoclonal antibodies MAB689-100, 621, eBioAITR, 110416.

More particularly, the antibodies useful for detecting Foxp3 are human anti-Foxp3 antibodies. Examples of antibodies which bind the human Foxp3 antigen include, without being limited to, the monoclonal antibodies PCH101, 259D, 206D, and 236A/E7.

More particularly, the antibodies useful for detecting TGFβ-1 are human anti-TGFβ-1 antibodies. Examples of antibodies which bind the human TGFβ-1 antigen include, without being limited to, the monoclonal antibodies MAB2463, TW4-6H10, 19D8, and eBio16TFB.

In one embodiment of the invention, at step (b) the means useful for isolating a population of IFNγ⁺IL-10⁺IL-34⁺ secreting human GITR⁺CD8⁺CD45RC^{low} Treg cells are antibodies useful for isolating a population of IFNγ⁺IL-10⁺IL-34⁺ secreting human CD8⁺CD45RC^{low} Treg cells as described hereinabove and antibodies useful for detecting IL-2.

More particularly, the antibodies useful for detecting IL-2 are human anti-IL-2 antibodies. Examples of antibodies which bind the human IL-2 antigen include, without being limited to, the monoclonal antibodies MQ1-17H12, and 5344.111.

In one embodiment, step (a) and step (b) of the method of the invention are carried out concomitantly. In other words, in one embodiment, the method of the invention for detecting and/or isolating IFNγ⁺IL-10⁺IL-34⁺ secreting human CD8⁺CD45RC^{low} Treg cells as described hereinabove from a biological sample containing human peripheral blood mononuclear cells (PBMCs) or lymphocytes comprises contacting said population of human PBMCs or lymphocytes with means useful for isolating a population of human IFNγ⁺IL-10⁺IL-34⁺ secreting human CD8⁺CD45RC^{low} Treg. Examples of suitable means are described hereinabove.

Also described is an in vitro or ex vivo method for generating a population of CD8⁺CD45RC^{low} Tregs according to the invention, comprising a step of culturing a population of CD8⁺ T cells with a culture medium comprising an isolated peptide derived from a MHC class II molecule, or a functional fragment thereof, in the presence of a population of dendritic cells, preferably of plasmacytoid dendritic cells (pDCs).

The isolated peptide derived from a MHC class II molecule may have a length ranging between 5 and 40 amino acids and comprise at least 5 contiguous amino acids from the amino acid sequence: REEYARFDSDVGEYR (SEQ ID NO: 1) or a function-conservative variant thereof. Preferably, the isolated peptide derived from a MHC class II molecule has a length ranging between 15 and 40 amino acids and comprises the amino acid sequence: REEYARFDSDVGEYR (SEQ ID NO: 1) or a function-conservative variant thereof.

Alternatively, the isolated peptide derived from a MHC class II molecule may have a length ranging between 11 and 16 amino acids and comprise the amino acid sequence: SDVGEYR (SEQ ID NO: 18) or a function-conservative variant thereof.

According to the present invention, the term "function-conservative variant" refers to peptides which comprises at least about 70% identity with a peptide of reference (such as SEQ ID NO: 1 or SEQ ID NO: 18), even more preferably at least about 75%, 80%, 85%, 90%, 95%, 96%, 97%, 98%, 99%, 99.5% or 99.9%, and which are still functional, i.e. still able to induce the generation of a population of CD8⁺CD45RC^{low} Tregs in substantially the same way as a peptide of reference (such as SEQ ID NO: 1 or SEQ ID NO: 12).

Preferably, said peptide derived from a MHC class II molecule is selected from the group consisting of:
REEYARFDSDVGEYR (SEQ ID NO: 1);
NREEYARFDSDVGEYR (SEQ ID NO: 2);
REEYARFDSDVGEFR (SEQ ID NO: 3);
REEYVRFDSDVGEYR (SEQ ID NO: 4);
QEEYARFD SD VGEYR (SEQ ID NO: 5);
REEYARFDSDVGVYR (SEQ ID NO: 6);
NREEYARFDSDVGEFR (SEQ ID NO: 7);
NREEYVRFDSDVGEYR (SEQ ID NO: 8);
NQEEYARFDSDVGEYR (SEQ ID NO: 9);
NREEYARFDSDVGVYR (SEQ ID NO: 10);
RLLARLIYNREEYARFDSDVGEYRAVTELGRPSAEYRNKQ (SEQ ID NO: 11);
YLRYDSDVGEYRAVTE (SEQ ID NO: 12);
DSDVGEYRAVTELGRP (SEQ ID NO: 13);
YLRYDSDVGEYR (SEQ ID NO: 14);
YLRYDSDVGEYRAV (SEQ ID NO: 15);
SDVGEYRAVTELGR (SEQ ID NO: 16);
LRYDSDVGEYRAVTE (SEQ ID NO: 17).

In one embodiment, said method comprises a step of culturing a population of CD8⁺ T with a culture medium comprising a MHC/peptide multimer comprising a peptide derived from a MHC class II molecule as described above.

According to the present invention, the term "MHC/peptide multimer" refers to a stable multimeric complex composed of major histocompatibility complex (MHC) protein subunits loaded with a peptide derived from a MHC class II molecule as described above. Said MHC/peptide multimers (also called herein MHC/peptide complex) include, but are not limited to, a MHC/peptide dimer, trimer, tetramer or pentamer. Preferably said MHC/peptide multimers are tetramers.

The peptides derived from a MHC class II molecule described and the MHC/peptide multimers are described in WO2015150491 and in WO2015150492.

### Method for enriching /Method for depleting / Kit

Also described is a method for enriching a cell population in IFNγ⁺IL-10⁺IL-34⁺ secreting human CD8⁺CD45RC^{low} Treg cells as described hereinabove, comprising:
- detecting the CD8⁺CD45RC^{low} Treg cells secreting IFNγ⁺IL-10⁺IL-34⁺ in a biological sample containing human peripheral blood mononuclear cells (PBMCs) or lymphocytes as described hereinabove,
- selecting said cells,
thereby obtaining a cell population enriched in IFNγ⁺IL-10⁺IL-34⁺ secreting human CD8⁺CD45RC^{low} Treg cells.

Thus, the method for enriching a population in IFNγ⁺IL-10⁺IL-34⁺ secreting human CD8⁺CD4SRC^{low} Treg cells as described hereinabove may comprise:
- contacting a biological sample containing human peripheral blood mononuclear cells (PBMCs) or lymphocytes with means useful for isolating a population of human CD8⁺CD45RC^{low} Treg cells,
- detecting and selecting the IFNγ⁺IL-10⁺IL-34⁺ secreting human CD8⁺CD45RC^{low} Treg cells from the CD8⁺CD45RC^{low} Treg cells,
thereby obtaining a cell population enriched in IFNγ⁺IL-10⁺IL-34⁺ secreting human CD8⁺CD45RC^{low} Treg cells.

Methods for detecting and/or selecting CD8⁺CD45RC^{low} Treg cells secreting IFNγ⁺IL-10⁺IL-34⁺ are described hereinabove. For example, methods for separating, isolating, or selecting cells include, but are not limited to magnetic separation using antibody-coated magnetic beads (Schwartz, et al, U.S. Pat. No. 5,759,793) and affinity chromatography or "panning" using antibody attached to a solid matrix (e.g. a plate). Further techniques providing accurate separation include fluorescence-activated cell sorters (FACS), which can have varying degrees of sophistication, such as having multiple color channels, low angle and obtuse light scattering detecting channels, or impedance channels. Dead cells can be eliminated by selection with dyes associated with dead cells e.g., (propidium iodide, LDS). Red blood cells can be removed by, for example, elutriation, hemolysis, or Ficoll-Paque gradients. Any technique well known in the art can be employed that is not unduly detrimental to the viability of the selected cells.

A method for enriching a population in IFNγ⁺IL-10⁺IL-34⁺ secreting human CD8⁺CD45RC^{low} Treg cells may comprise:
- contacting a biological sample containing human peripheral blood mononuclear cells (PBMCs) or lymphocytes with at least one reagents which binds to a marker of non-CD8⁺ cells, thereby depleting non-CD8⁺ cells,
- detecting in IFNγ⁺IL-10⁺IL-34⁺ secreting human CD8⁺CD45RC^{low} Treg cells as described hereinabove and selecting them,
thereby obtaining a cell population enriched in IFNγ⁺IL-10⁺IL-34⁺ secreting human CD8⁺CD45RC^{low} Treg cells.

The method for enriching a population in IFNγ⁺IL-10⁺IL-34⁺ secreting human CD8⁺CD45RC^{low} Treg cells may comprise:
- contacting a biological sample containing human peripheral blood mononuclear cells (PBMCs) or lymphocytes with at least one reagents which binds to a marker of non-CD8⁺ cells, thereby depleting non-CD8⁺ cells,
- detecting and isolating the CD8⁺CD45RC^{low} Treg cells as described hereinabove, and
- detecting and selecting the IFNγ⁺IL-10⁺IL-34⁺ secreting human CD8⁺CD45RC^{low} Treg cells as described hereinabove from the CD8⁺CD45RC^{low} Treg cells,
thereby obtaining a cell population enriched in IFNγ⁺IL-10⁺IL-34⁺ secreting human CD8⁺CD45RC^{low} Treg cells.

Examples of markers that bind to non-CD8⁺ cells include, but are not limited to, CD4, CD14, CD16, CD19.

Preferred reagents that bind to said markers are antibodies. In one embodiment, the antibodies are conjugated to a fluorochrome or magnetic particle. In another embodiment, the cell selection is performed by flow cytometry, fluorescence activated cell sorting, magnetic selection, affinity chromatography or panning or combinations thereof.

Also described is an enriched population of IFNγ⁺IL-10⁺IL-34⁺ secreting human CD8⁺CD45RC^{low} Treg cells. Said enriched population of IFNγ⁺IL-10⁺IL-34⁺ secreting human CD8⁺CD45RC^{low} Treg cells may be obtainable by a method for enriching a population in IFNγ⁺IL-10⁺IL-34⁺ secreting human CD8⁺CD45RC^{low} Treg cells as described hereinabove.

As used herein, an enriched population of IFNγ⁺IL-10⁺IL-34⁺ secreting human CD8⁺CD45RC^{low} Treg cells is one in which the percentage of IFNγ⁺IL-10⁺IL-34⁺ secreting human CD8⁺CD45RC^{low} Treg cells is higher than the percentage of IFNγ⁺IL-10⁺IL-34⁺ secreting human CD8⁺CD45RC^{low} Treg cells in the originally obtained population of cells. Although possible, an enriched population of IFNγ⁺IL-10⁺IL-34⁺ secreting human CD8⁺CD45RC^{low} Treg cells need not contain a homogenous population of IFNγ⁺IL-10⁺IL-34⁺ secreting human CD8⁺CD45RC^{low} Treg cells. In particular embodiments, at least about 50%, about 55%, about 60%, about 65%, about 70%, about 75%, about 80%, about 85%, about 90%, about 95%, about 98%, or about 99% of said cells of the composition are IFNγ⁺IL-10⁺IL-34⁺ secreting human CD8⁺CD45RC^{low} Treg cells. In another embodiment, the percentage of IFNγ⁺IL-10⁺IL-34⁺ secreting human CD8⁺CD45RC^{low} Treg cells in the enriched population is at least twice, 5 times, 10 times, 20 times, 30 times, 40 times, 50 times, 60 times, 70 times, 80 times, 90 times, 100 times the percentage of IFNγ⁺IL-10⁺IL-34⁺ secreting human CD8⁺CD45RC^{low} Treg cells before enrichment.

Enrichment methods are variable based on the level of enrichment associated with each step of the enrichment process. The level of enrichment and percent purity of the IFNγ⁺IL-10⁺IL-34⁺ secreting human CD8⁺CD45RC^{low} Treg cells will depend on many factors including, but not limited to, the donor, the cell/tissue source and the disease state of the donor. In a particular embodiment, the IFNγ⁺IL-10⁺IL-34⁺ secreting human CD8⁺CD45RC^{low} Treg cells are enriched at least about 2-fold, about 5-fold, about 10-fold, about 15-fold, about 20-fold, about 25-fold, about 30-fold, about 35-fold, about 40-fold, about 50-fold, about 55-fold, about 60-fold, about 65-fold, about 70-fold, about 75-fold, about 80-fold, about 85-fold, about 90-fold, about 95-fold, about 100-fold, about 105-fold, about 110-fold, about 115-fold, about 120-fold, about 130-fold, about 140-fold, about 150-fold, or about 200-fold.

Also described is a method for depleting a cell population in IFNγ⁺IL-10⁺IL-34⁺ secreting human CD8⁺CD45RC^{low} Treg cells, comprising:
- detecting the CD8⁺CD45RC^{low} Treg cells secreting IFNγ⁺IL-10⁺IL-34⁺ in a biological sample containing human peripheral blood mononuclear cells (PBMCs) or lymphocytes as described hereinabove,
- depleting said cells,
- thereby obtaining a cell population depleted in IFNγ⁺IL-10⁺IL-34⁺ secreting human CD8⁺CD45RC^{low} Treg cells.

Methods for detecting and/or selecting CD8⁺CD45RC^{low} Treg cells secreting IFNγ⁺IL-10⁺IL-34⁺ are described hereinabove.

Also described is an IFNγ⁺IL-10⁺IL-34⁺ secreting human CD8⁺CD45RC^{low} Treg cell-depleted population. Said IFNγ⁺IL-10⁺IL-34⁺ secreting human CD8⁺CD45RC^{low} Treg cell-depleted population may be obtainable according to a method for depleting a cell population in IFNγ⁺IL-10⁺IL-34⁺ secreting human CD8⁺CD45RC^{low} Treg cells as described hereinabove.

As used herein, an IFNγ⁺IL-10⁺IL-34⁺ secreting human CD8⁺CD45RC^{low} Treg cell-depleted population is one in which the percentage of IFNγ⁺IL-10⁺IL-34⁺ secreting human CD8⁺CD45RC^{low} Treg cells is lower than the percentage of IFNγ⁺IL-10⁺IL-34⁺ secreting human CD8⁺CD45RC^{low} Treg cells in the originally obtained population of cells.

In one embodiment, the percentage of IFNγ⁺IL-10⁺IL-34⁺ secreting human CD8⁺CD45RC^{low} Treg cells in the depleted population is at most 0.5 times, 0.4 times, 0.3 times, 0.25 times, 0.2 times, 0.15 times, 0.1 times the percentage of IFNγ⁺IL-10⁺IL-34⁺ secreting human CD8⁺CD45RC^{low} Treg cells before depletion.

Depletion of regulatory T cells has been shown to enhance vaccine-mediated immunity in cancer patients (Dannull et al., 2005, 115(12):3623-3633). Thus, it is proposed that depletion of the IFNγ⁺IL-10⁺IL-34⁺ secreting human CD8⁺CD45RC^{low} Treg cells of the invention may be used in cell therapy for treating cancer or infectious disease wherein said Treg cells may be deleterious or harmful for the treatment. For example, in cancer treatment, a transient depletion of IFNγ⁺IL-10⁺IL-34⁺ secreting human CD8⁺CD45RC^{low} Treg cells could be interesting before a vaccination/immunotherapy protocol, said transient depletion being obtained by depleting the blood of a patient ex vivo through a device according to the depletion method and re-injecting it immediately to the patient. Thus, in said embodiment, the depletion of IFNγ⁺IL-10⁺IL-34⁺ secreting human CD8⁺CD45RC^{low} Treg cells from the blood of the patient is carried out ex vivo through a device.

Also described is a kit for identifying or isolating an IFNγ⁺IL-10⁺IL-34⁺ secreting human CD8⁺CD45RC^{low} Treg cell population, or for enriching or depleting a cell population in IFNγ⁺IL-10⁺IL-34⁺ secreting human CD8⁺CD45RC^{low} Treg cells, said kit comprising reagents for detecting the cell surface expression of CD3, CD8, CD45RC and means for detecting secretion of IFNγ and IL-10 and/or IL-34.

Preferably, said reagents are antibodies. More preferably, these antibodies are conjugated to a fluorochrome or a magnetic particle.

In one particular embodiment, said reagents are antibodies conjugated to a cytotoxic drug such as anti-microtubule agents, alkylating agents and DNA minor groove binding agents. Examples of cytotoxic drugs include, without being limited to, mitomycin, auristatins and maytansine derivatives.

In another embodiment, said reagents are antibodies conjugates to antibiotics. Example of antibiotics include, without being limited to, chloramphenicol, erythromycin, lincomycin, fusidic acid, streptomycin, an aminoglycoside antibiotic, a tetracycline, a polymyxin, fosfomycin, vancomycin, ristocetin, bacitracin, gramacidin, a penicillin, and a cephalosporin
In one embodiment, said kit further comprises reagents for detecting Foxp3 expression and/or GITR expression and/or TGFβ-1 expression and/or IL-2 expression. Preferably, said reagents are antibodies. More preferably, said antibodies are conjugated to a fluorochrome.

### Means for expanding the Treg cells of the invention:

In a third aspect, the invention relates to a method for expanding IFNγ⁺IL-10⁺IL-34⁺ secreting human CD8⁺CD45RC^{low} Treg cells as described hereinabove comprising a step of culturing an isolated population of IFNγ⁺IL-10⁺IL-34⁺ secreting human CD8⁺CD45RC^{low} Treg cells, preferably a step of contacting an isolated population of IFNγ⁺IL-10⁺IL-34⁺ secreting human CD8⁺CD45RC^{low} Treg cells with a culture medium suitable for expanding Treg cells.

In one embodiment, the invention relates to a method for expanding IFNγ⁺IL-10⁺IL-34⁺ secreting human CD8⁺CD45RC^{low} Treg cells, said Treg cells optionally being GITR⁺ and/or Foxp3⁺, comprising a step of contacting an isolated population of IFNγ⁺IL-10⁺IL-34⁺ secreting human CD8⁺CD45RC^{low} Treg cells with a culture medium suitable for expanding Treg cells.

In one embodiment, the invention relates to a method for expanding IFNγ⁺IL-10⁺IL-34⁺ secreting human CD8⁺CD45RC^{low} Treg cells expressing at least one of GITR, Foxp3 and TGFβ-1 comprising a step of contacting an isolated population of IFNγ⁺IL-10⁺IL-34⁺ secreting human GITR⁺CD8⁺CD45RC^{low} Treg cells with a culture medium suitable for expanding Treg cells.

In one embodiment, the isolated population of IFNγ⁺IL-10⁺IL-34⁺ secreting human CD8⁺CD45RC^{low} Treg cells of the invention is frozen and subsequently thawed before being contacted with a culture medium suitable for expanding Treg cells. The inventors demonstrated that the IFNγ⁺IL-10⁺IL-34⁺ secreting human CD8⁺CD45RC^{low} Tregs of the invention can be efficiently expanded, even more so after thawing. Indeed, the inventors observed that the IFNγ⁺EL-10⁺IL-34⁺ secreting human CD8⁺CD45RC^{low} Tregs of the invention proliferated more efficiently when thawed than when freshly sorted.

As used herein, the term "expanding" refers to the process of converting and/or amplifying a given population of cells (e.g. immune cells such as T cells). Expansion of T cells is preferably performed by culturing a cell population comprising T cells in the presence of antigen-specific stimulating agent such as antigens, cells (such as donor's cells within the context of allograft transplantation), MHC polymers, antibodies (such as anti-CD3 antibodies and/or anti-CD28 antibodies), lectins (such as PHA), etc. Expansion may also require culture of T cells in the presence of a cytokine (such as IL-15, IL-12, IL-4, IL-7, IL-2, IFNγ, IL-34 and pro-inflammatory cytokines (such as, for example, IL-1 (in particular IL-1β), IL-6 and TNFα)).

As used herein, the term "culture medium" refers to a medium for maintaining a cell population, or culturing a cell population containing nutrients that maintain cell viability and support proliferation. The culture medium may contain any of the following in an appropriate combination: salt(s), buffer(s), amino acids, glucose or other sugar(s), antibiotics, serum or serum replacement, and other components such as growth factors, cytokines etc. Culture media ordinarily used for particular cell types are known to those skilled in the art. The culture medium of the invention may be based on a commercially available medium such as RPMI 1640 from Invitrogen.

In one embodiment, the medium suitable for expanding the human CD8⁺CD45RC^{low} Tregs of the invention comprises at least one stimulating agent. In one embodiment, said at least one stimulating agent is selected from the group comprising antigens, cells, MHC polymers, antibodies and lectins. In one embodiment, said at least one stimulating agent is antigen-specific and is selected from the group consisting of antigens, cells, MHC polymers and antibodies. In one embodiment, said at least one stimulating agent is selected from the group comprising allogeneic antigen presenting cells, anti-CD3 antibodies and/or anti-CD8 antibodies, PMA + ionomycin.

In one embodiment, the medium suitable for expanding the human CD8⁺CD45RC^{low} Tregs of the invention comprises an amount of at least one cytokine. Examples of cytokines that may be present in the medium suitable for expanding Treg include, but are not limited to, IL-15, IL-12, IL-4, IL-7, IL-2, IFNγ, IL-34 and pro-inflammatory cytokines (such as, for example, IL-1 (in particular IL-1β), IL-6 and TNFα).

In one embodiment, the culture medium suitable for expanding Treg cells comprises an amount of interleukin-2 (IL-2) and/or an amount of interleukin-15 (IL-15).

In a particular embodiment, IL-2 is a human interleukin-2 (hIL-2), preferably a recombinant human interleukin-2 (rhIL-2). It should be further noted that rhIL-2 is commercially available for pharmaceutical uses. Suitable commercial forms include, e.g. Proleukin®, a recombinant human IL-2 composition.

In a particular embodiment, IL-15 is a human interleukin-15 (hIL-15), preferably a recombinant human interleukin-15 (rhIL-15).

Typically, IL-2 is added to the culture medium at a concentration ranging from 100 to 10000 U/ml, preferably from 500 to 5000 U/ml, more preferably at 1000 U/ml.

Typically, IL-15 is added to the culture medium at a concentration ranging from 1 to 100 ng/ml, preferably from 2.5 to 50 ng/ml, more preferably at 10 ng/ml.

Typically, IL-12 is added to the culture medium at a concentration ranging from 0.01 to 500 ng/ml, preferably from 1 to 50 ng/ml, more preferably at 5 ng/ml.

Typically, IL-4 is added to the culture medium at a concentration ranging from 0.01 to 500 ng/ml, preferably from 1 to 20 ng/ml, more preferably at 5 ng/ml.

Typically, IL-7 is added to the culture medium at a concentration ranging from 0.01 to 500 ng/ml, preferably from 2.5 to 50 ng/ml, more preferably at 10 ng/ml.

Typically, IFNγ is added to the culture medium at a concentration ranging from 0.01 to 500 ng/ml, preferably from 5 to 100 ng/ml, more preferably at 20 ng/ml.

Typically, IL-34 is added to the culture medium at a concentration ranging from 0.01 to 500 ng/ml, preferably from 10 to 100 ng/ml, more preferably at 50 ng/ml.

Typically, IL-1 is added to the culture medium at a concentration ranging from 0.01 to 500 ng/ml, preferably from 2.5 to 50 ng/ml, more preferably at 10 ng/ml.

Typically, IL-6 is added to the culture medium at a concentration ranging from 0.01 to 500 ng/ml, preferably from 5 to 100 ng/ml, more preferably at 20 ng/ml.

Typically, TNF is added to the culture medium at a concentration ranging from 0.01 to 500 ng/ml, preferably from 5 to 100 ng/ml, more preferably at 20 ng/ml.

Typically, TGFbeta is added to the culture medium at a concentration ranging from 0.01 to 500 ng/ml, preferably from 0.1 to 10 ng/ml, more preferably at 1 ng/ml.

Typically, the culture of the human CD8⁺CD45RC^{low} Treg cells of the invention with a culture medium of interest shall be carried out for between at least 6 days, such as, for example, between at least 6 days and no more than 15 days, or between at least 6 days and no more than 20 days, or between at least 6 days and no more than 6 to 8 weeks preferably 14 days. In one embodiment, the culture of the human CD8⁺CD45RC^{low} Treg cells of the invention with a culture medium of interest is carried out for 6, 7, 8, 9, 10, 11, 12, 13, 14, 15, 16, 17, 18, 19, 20, 21, 22, 23, 24, 25, 26, 27, 28, 29, 30 or 31 days. In one embodiment, the culture of the human CD8⁺CD45RC^{low} Treg cells of the invention with a culture medium of interest is carried out for 1 week, 2 weeks, 3, 4, 5, 6, 7, 8, 9 or 10 weeks or more.

In one embodiment, cytokines, preferably IL-2 and/or IL-15, are added to the culture medium at day 0 of culture of the human CD8⁺CD45RC^{low} Treg cells of the invention. In another embodiment, cytokines, preferably IL-2 and/or IL-15, are further added to the culture medium once, twice or three or four times or more, for example at day 1, 2, 3, 4, 5, 6, 7, 8, 9, 10, 11, 12, 13, 14, 15, 16, 17, 18, 19 and/or 20. In one embodiment, cytokines, preferably IL-2 and/or IL-15, are added to the culture medium at day 0 and at day 5, 6, 7, 8 or 9 of culture of the human CD8⁺CD45RC^{low} Treg cells of the invention, preferably at days 0 and 7. In another embodiment, cytokines, preferably IL-2 and/or IL-15 are added to the culture medium three times, preferably at day 0 and two additional times. In another embodiment, cytokines, preferably IL-2 and/or IL-15, are added to the culture medium four times, preferably at day 0 and three additional times. In another embodiment, cytokines, preferably IL-2 and/or IL-15, are added to the culture medium 5 times, preferably at day 0 and 4 additional times, such as, for example, at days 0, 6, 13, 16 and 18. In one embodiment, cytokines, preferably IL-2 and/or IL-15, are added at day 0 and every 2, 3 or 4 days until the end of the culture

In one embodiment, the culture medium suitable for expanding Treg cells comprises an amount of at least one antigen of interest. Typically, a given antigen is added to the culture medium at a concentration of 1µg/ml.

In a particular embodiment, the expansion is a polyclonal expansion. A polyclonal expansion may be obtained by using anti-CD3 Ab and anti-CD28 Ab and/or allogeneic antigen-presenting cells (APCs).

In one embodiment, 0.1 to 10 µg/ml, preferably 0.25 to 4 µg/ml, more preferably 1 µg/ml of anti-CD3 antibody and/or 0.1 to 10 µg/ml, preferably 0.25 to 4 µg/ml, more preferably 1 µg/ml of anti-CD28 antibody are added to the culture medium.

In one embodiment, allogeneic antigen-presenting cells (APCs) at a Tregs:APCs ratio ranging from 1:1 to 1:10, preferably from 1:2 and 1:6, and more preferably of 1:4 are added to the culture medium.

Within the context of the invention, the isolated population of IFNγ⁺IL-10⁺IL-34⁺ secreting human CD8⁺CD45RC^{low} Treg cells as described hereinabove, expanded or not, may be then pulsed with an antigen of interest in order to achieve a population of antigen-specific IFNγ⁺IL-10⁺IL-34⁺ secreting human CD8⁺CD45RC^{low} Treg cells, said antigen being provided in an amount effective to "prime" the isolated population of IFNγ⁺IL-10⁺IL-34⁺ secreting human CD8⁺CD45RC^{low} Treg cells of the invention and thus obtain a population of IFNγ⁺IL-10⁺IL-34⁺ secreting human CD8⁺CD45RC^{low} Treg cells specific for said antigen. Indeed, such expanded antigen-specific IFNγ⁺IL-10⁺IL-34⁺ secreting human CD8⁺CD45RC^{low} Treg cells are useful in the prevention or treatment of unwanted immune responses, such as those involved in autoimmune disorders, immune reactions to therapeutic proteins, and/or allergies.

Accordingly, populations of IFNγ⁺IL-10⁺IL-34⁺ secreting human CD8⁺CD45RC^{low} Treg cells specific for an antigen associated with the disease to be treated (pathogenic antigen) should be obtained. Therefore, the antigen of interest is selected from the group consisting of auto-antigens, allo-antigens, food antigens from common human diet, inflammatory antigens and allergens.

The term "auto-antigen" refers to an immunogenic peptide derived from a protein of an individual. It may be, by way of example, an auto-antigen of the following non-limiting list: acetylcholine receptor, actin, adenine nucleotide translocator, adrenoreceptor, aromatic L-amino acid decarboxylase, asialoglycoprotein receptor, bactericidal/permeability increasing protein (BPi), calcium sensing receptor, cholesterol side chain cleavage enzyme, collagen type IV-chain, cytochrome P450 2D6, desmin, desmoglein-1, desmoglein-3, F-actin, GM-gangliosides, glutamate decarboxylase, glutamate receptor, H/K ATPase, 17-α-hydroxylase, 21-hydroxylase, IA-2 (ICAS12), insulin, insulin receptor, intrinsic factor type 1, leucocyte function antigen 1, myelin associated glycoprotein, myelin basic protein, myelin oligodendrocyte protein, myosin, P80-coilin, pyruvate deshydrogenase complex E2 (PDC-E2), sodium iodide symporter, SOX-10, thyroid and eye muscle shared protein, thyroglobulin, thyroid peroxidase, thyrotropin receptor, tissue transglutaminase, transcription coactivator p75, tryptophan hydroxylase, tyrosinase, tyrosine hydroxylase, ACTH, aminoacyl-tRNA-histidyl synthetase, cardiolipin, carbonic anhydrase II, centromere associated proteins, DNA-dependent nucleosome-stimulated ATPase, fibrillarin, fibronectin, glucose 6 phosphate isomerase, beta 2-glycoprotein I, golgin (95, 97, 160, 180), heat shock proteins, hemidesmosomal protein 180, histone H2A, H2B, keratin, IgE receptor, Ku-DNA protein kinase, Ku-nucleoprotein, La phosphoprotein, myeloperoxidase, proteinase 3, RNA polymerase I-III, signal recognition protein, topoisomerase I, tubulin, vimentin, myelin associated oligodendrocyte basic protein (MOBP), proteolipid protein, oligodendrocyte specific protein (OSP/Claudin 11), 2',3'-cyclic nucleotide 3 -phosphodiesterase (CNPase), BP antigen 1 (BPAG1-e), transaldolase (TAL), human mitochondrial autoantigens PDC-E2 (Novo 1 and 2), OGDC-E2 (Novo 3), and BCOADC-E2 (Novo 4), bullous pemphigoid (BP)180, laminin 5 (LN5), DEAD-box protein 48 (DDX48) or insulinoma-associated antigen-2.

The term "food antigen from common human diet" refers to an immunogenic peptide, which comes from foodstuffs common for humans, such as food antigens of the following non-limiting list: bovine antigens such as lipocalin, Ca-binding S100, alpha-lactalbumin, lactoglobulins such as beta-lactoglobulin, bovine serum albumin, caseins. Food-antigens may also be atlantic salmon antigens such as parvalbumin, chicken antigens such as ovomucoid, ovalbumin, Ag22, conalbumin, lysozyme or chicken serum albumin, peanuts, shrimp antigens such as tropomyosin, wheat antigens such as agglutinin or gliadin, celery antigens such as celery profilin, carrot antigens such as carrot profilin, apple antigens such as thaumatin, apple lipid transfer protein, apple profilin, pear antigens such as pear profilin, isoflavone reductase, avocado antigens such as endochitinase, apricot antigens such as apricot lipid transfer protein, peach antigens such as peach lipid transfer protein or peach profilin, soybean antigens such as HPS, soybean profilin or (SAM22) PR-10 prot.

The term "inflammatory antigen" refers to myelin basic protein (MBP), myelin associated glycoprotein (MAG), myelin oligodendrocyte glycoprotein (MOG), proteolipid protein (PLP), oligodendrocyte myelin oligoprotein (OMGP), myelin associated oligodendrocyte basic protein (MOBP), oligodendrocyte specific protein (OSP/Claudin 1), heat shock proteins, oligodendrocyte specific proteins (OSP), NOGO A, glycoprotein Po, peripheral myelin protein 22 (PMP22), 2',3'-cyclic nucleotide 3 -phosphodiesterase (CNPase), citrulline-substituted cyclic and linear filaggrin peptides, type II collagen peptides, human cartilage glycoprotein 39 (HCgp39) peptides, HSP, heterogeneous nuclear ribonucleoprotein (hnRNP) A2 peptides, hnRNP B1, hnRNP D, Ro60/52, HSP60, 65, 70 and 90, BiP, keratin, vimentin, fibrinogen, collagen type I, III, IV and V peptides, annexin V, Glucose 6 phosphate isomerase (GPI), acetyl-calpastatin, pyruvate deshydrogenase (PDH), aldolase, topoisomerase I, snRNP, PARP, Scl-70, Scl-100, phospholipid antigen including anionic cardiolipin and phosphatidylserine, neutrally charged phosphatidylethanolamine and phosphatidylcholine, matrix metalloproteinase, fibrillin, aggreccan.

The term "allergen" refers to an inhaled allergen, an ingested allergen or a contact allergen. Examples of allergens include, but are not limited to, inhaled allergens derived from pollens (Cup, Jun), house dust mites (Der, Gly, Tyr, Lep), dog, cat and rodents (Can, Fel, Mus, Rat). Examples of contact allergens include, but are not limited to, heavy metals (such as nickel, chrome, gold), latex, haptens such as halothane, hydralazine.

Thus, in one embodiment, when the autoimmune disease is multiple sclerosis, the autoantigen is selected from the group consisting of myelin-related antigens (e.g. myelin basic protein (MBP) (e.g. MBP83-102 peptide), myelin oligodendrocyte glycoprotein (MOG) (e.g. MOG35-55 peptide) and proteolipid protein (PLP) (e.g. PLP139-151 peptide). When the autoimmune disease is Type I diabetes (T1D), the autoantigen is selected from the group consisting of insulin, insulin precursor proinsulin (ProIns), glutamic acid decarboxylase 65 (GAD65), glial fibrillary acidic protein (GFAP), islet-specific glucose-6-phosphatase catalytic subunit-related protein (IGRP), insulinoma-associated antigen-2 (IA-2) and zinc transporter 8 (ZnT8). When the autoimmune disease is rheumatoid arthritis, the autoantigen is type II collagen (CTII).

It is also intended that alloantigens include, but are not limited to, antigens expressed by the allograft, proteins expressed in the course of gene therapy (and also viral antigens issued from the viral vector used) as well as therapeutic proteins. The alloantigen is selected from the group consisting of HLA-A, HLA-B, HLA-C, HLA-E, HLA-F, HLA-G, HLA-DM, HLA-DO, HLA-DP, HLA-DQ, HLA-DR, minor antigens such as HA-1, HA-2, HA-3, HA-8, HB-1, UGT2B17, BCL2A1, HY B7, HY A2, HY A1, HY B60, HY B8, HY DQ5, HY DRB3, and blood group antigens A and B.

As such an "allograft" is a transplant between two individuals of the same species having two genetically different MHC haplotypes but also minor histocompatibility antigens and blood groups. Thus, for preventing or treating allograft rejection, the IFNγ⁺IL-10⁺IL-34⁺ secreting human CD8⁺CD45RC^{low} Treg cells are typically isolated from the patient (or recipient) and expanded by using cells from the donor. Alternatively, the IFNγ⁺IL-10⁺IL-34⁺ secreting human CD8⁺CD45RC^{low} Treg cells may be expanded by using a mix of antigens obtained from the donor.

The term "therapeutic proteins" refers to proteins or peptides and their administration in the therapy of any given condition or illness. Therapeutic proteins relate to any protein or peptide, such as therapeutic antibodies, cytokines, enzymes or any other proteins, that is administered to a patient. Examples of protein therapy relate to treatment of hemophilia via administration of plasma-derived or recombinant clotting factor concentrates (e.g. factor VIII and factor IX), the treatment of cancer or cardiovascular diseases using monoclonal antibodies or the treatment of metabolic or lysosomal diseases by enzyme replacement therapy.

In a particular embodiment, the culture medium suitable for expanding Treg cells comprises an amount of interleukin-2 (IL-2) and/or an amount of interleukin-15 (IL-15) and an amount of at least one antigen of interest.

Also described is an isolated population of expanded IFNγ⁺IL-10⁺IL-34⁺ secreting human CD8⁺CD45RC^{low} Treg cells, an isolated population of expanded IFNγ⁺IL-10⁺IL-34⁺ secreting human GITR⁺CD8⁺CD45RC^{low} Treg cells, an isolated population of expanded IFNγ⁺IL-10⁺IL-34⁺ secreting human Foxp3⁺CD8⁺CD45RC^{low} Treg cells, an isolated population of expanded TGFβ-1⁺IFNγ⁺IL-10⁺IL-34⁺ secreting human CD8⁺CD45RC^{low} Treg cells, an isolated population of expanded IFNγ⁺IL-10⁺IL-34⁺ secreting human CD8⁺CD45RC^{low} Treg cells, wherein said Treg cells are GITR⁺ and/or Foxp3⁺, an isolated population of expanded IFNγ⁺IL-10⁺IL-34⁺ secreting human Foxp3⁺GITR⁺CD8⁺CD45RC^{low} Treg cells, an isolated population of expanded TGFβ-1⁺IFNγ⁺IL-10⁺IL-34⁺ secreting human Foxp3⁺CD8⁺CD45RC^{low} Treg cells, an isolated population of expanded TGFβ-1⁺IFNγ⁺IL-10⁺IL-34⁺ secreting human GITR⁺CD8⁺CD45RC^{low} Treg cells and an isolated population of expanded TGFβ-1⁺IFNγ⁺IL-10⁺IL-34⁺ secreting human Foxp3⁺GITR⁺CD8⁺CD45RC^{low} Treg cells.

Said isolated populations of expanded IFNγ⁺IL-10⁺IL-34⁺ secreting human CD8⁺CD45RC^{low} Treg cells as described above may be obtainable by a method for expanding the Treg cells of the invention as above-described. In a particular embodiment, said IFNγ⁺IL-10⁺IL-34⁺ secreting human CD8⁺CD45RC^{low} Treg cells are antigen-specific.

Also described is a method for producing a population of CD8⁺ Tregs comprising:
- isolating CD8⁺GITR⁺ Treg cells from a biological sample containing human peripheral blood mononuclear cells (PBMCs) or lymphocytes, optionally isolating CD8⁺CD45RC^{low}GITR⁺ Treg cells from a biological sample containing human peripheral blood mononuclear cells (PBMCs) or lymphocytes,
- optionally freezing and subsequently thawing the isolated Treg cells, and
- expanding said cells in culture.

In one embodiment, said method comprises:
- isolating CD8⁺GITR⁺ Treg cells from a biological sample containing human peripheral blood mononuclear cells (PBMCs) or lymphocytes, wherein the isolated CD8⁺GITR⁺ Treg cells are IFNγ⁺IL-10⁺IL-34⁺ secreting human CD8⁺CD45RC^{low} Treg cells,
- optionally freezing and subsequently thawing the isolated Treg cells, and
- expanding said cells in culture.

Indeed, the inventors demonstrated that the subpopulation of GITR⁺CD8⁺CD45RC^{low} Treg cells has a high suppressive activity.

Also described is a method for producing a population of IFNγ⁺IL⁻10⁺IL-34⁺ secreting human CD8⁺CD45RC^{low} Treg cells comprising:
- isolating CD8⁺GITR⁺ Treg cells from a biological sample containing human peripheral blood mononuclear cells (PBMCs) or lymphocytes, optionally isolating CD8⁺CD45RC^{low}GITR⁺ Treg cells from a biological sample containing human peripheral blood mononuclear cells (PBMCs) or lymphocytes,
- optionally freezing and subsequently thawing the isolated Treg cells, and
- expanding said cells in culture.

In one embodiment, said method comprises:
- isolating CD8⁺GITR⁺ Treg cells from a biological sample containing human peripheral blood mononuclear cells (PBMCs) or lymphocytes,
   optionally freezing and subsequently thawing the isolated Treg cells, and
- expanding said cells in culture.

In another embodiment, said method comprises:
- isolating CD8⁺CD45RC^{low}GITR⁺ Treg cells from a biological sample containing human peripheral blood mononuclear cells (PBMCs) or lymphocytes,
- optionally freezing and subsequently thawing the isolated Treg cells, and
- expanding said cells in culture.

Methods for isolating human CD8⁺ Tregs cells and for expanding Treg cells are described hereinabove.

In one embodiment, CD8⁺GITR⁺ Treg cells may be isolated with antibodies useful for detecting GITR. Examples of antibodies which bind the human GITR antigen include, without being limited to, the monoclonal antibodies MAB689-100, 621, eBioAITR, 110416.

In another embodiment, CD8⁺CD45RC^{low}GITR⁺ Treg cells may be isolated with antibodies useful for detecting GITR and antibodies useful for detecting CD45RC. Examples of antibodies which bind the human GITR antigen include, without being limited to, the monoclonal antibodies MAB689-100, 621, eBioAITR, 110416. Examples of antibodies which bind the human CD45RC antigen include the monoclonal antibodies MT2 and RP1/12.

In one embodiment, the CD8⁺GITR⁺ Treg cells or the CD8⁺CD45RC^{low}GITR⁺ Treg cells are isolated according to the method of the invention after activation of the PBMCs or lymphocytes with at least one stimulating agent. Examples of stimulated agents are described hereinabove.

In one embodiment, the IFNγ⁺IL-10⁺IL-34⁺ secreting human CD8⁺CD45RC^{low} Treg cells as described hereinabove are derived and isolated from a culture of induced pluripotent stem cells (iPSC) or iPSC derived CD34⁺ cells. Within the context of the invention, a culture of iPSC or iPSC derived CD34⁺ cells may be obtained, for example, from a biological sample containing human peripheral blood mononuclear cells (PBMCs).

Also described is a method for depleting a population of CD8⁺ Tregs according to the invention comprising:
- detecting CD8⁺GITR⁺ Treg cells in a biological sample containing human peripheral blood mononuclear cells (PBMCs) or lymphocytes, optionally detecting CD8⁺CD45RC^{low}GITR⁺ Treg cells in a biological sample containing human peripheral blood mononuclear cells (PBMCs) or lymphocytes, and
- depleting said cells,
- thereby obtaining a cell population depleted in IFNγ⁺IL-10⁺IL-34⁺ secreting human CD8⁺CD45RC^{low} Treg cells.

### Pharmaceutical compositions of the invention:

Also described is a composition comprising the population of IFNγ⁺IL-10⁺IL-34⁺ secreting human CD8⁺CD45RC^{low} Treg cells according to the invention, preferably the isolated population of IFNγ⁺IL-10⁺IL-34⁺ secreting human CD8⁺CD45RC^{low} Treg cells.

Also described is a composition that comprises an enriched population of CD8⁺ human Tregs, in particular of IFNγ⁺IL-10⁺IL-34⁺ secreting human CD8⁺CD45RC^{low} Treg cells. The composition may also comprise an IFNγ⁺IL-10⁺IL-34⁺ secreting human CD8⁺CD45RC^{low} Treg cell-depleted population. The composition may also comprise a population of genetically modified IFNγ⁺IL-10⁺IL-34⁺ secreting human CD8⁺CD45RC^{low} Treg cells.

The composition may also comprise an expanded population of IFNγ⁺IL-10⁺IL-34⁺ secreting human CD8⁺CD45RC^{low} Treg cells, in particular an expanded population of antigen-specific IFNγ⁺IL-10⁺IL-34⁺ secreting human CD8⁺CD45RC^{low} Treg cells.

The invention also provides a pharmaceutical composition comprising an isolated population of IFNγ⁺IL-10⁺IL-34⁺ secreting human CD8⁺CD45RC^{low} Treg cells according to the invention and a pharmaceutically acceptable carrier. Also described is a pharmaceutical composition that comprises an enriched population of IFNγ⁺IL-10⁺IL-34⁺ secreting human CD8⁺CD45RC^{low} Treg cells as described hereinabove and a pharmaceutically acceptable carrier, a pharmaceutical composition that comprises a population of genetically modified IFNγ⁺IL-10⁺IL-34⁺ secreting human CD8⁺CD45RC^{low} Treg cells as described hereinabove and a pharmaceutically acceptable carrier, a pharmaceutical composition that comprises a population of expanded IFNγ⁺IL-10⁺IL-34⁺ secreting human CD8⁺CD45RC^{low} Treg cells as described hereinabove and a pharmaceutically acceptable carrier, and a pharmaceutical composition that comprises a population of expanded antigen-specific IFNγ⁺IL-10⁺IL-34⁺ secreting human CD8⁺CD45RC^{low} Treg cells as described hereinabove and a pharmaceutically acceptable carrier.

The pharmaceutical composition may generally include one or more pharmaceutically acceptable and/or approved additives, antibiotics, preservatives, adjuvants, diluents and/or stabilizers. Such auxiliary substances can be water, saline, glycerol, ethanol, wetting or emulsifying agents, pH buffering substances, or the like. Suitable carriers are typically large, slowly metabolized molecules such as proteins, polysaccharides, polylactic acids, polyglycollic acids, polymeric amino acids, amino acid copolymers, lipid aggregates, or the like. This pharmaceutical composition can contain additional additives such as mannitol, dextran, sugar, glycine, lactose or polyvinylpyrrolidone or other additives such as antioxidants or inert gas, stabilizers or recombinant proteins (e. g. human serum albumin) suitable for *in vivo* administration.

As used herein, the term "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The invention also provides an isolated population of IFNγ⁺IL-10⁺IL-34⁺ secreting human CD8⁺CD45RC^{low} Treg cells according to the invention for use as a medicament. Also described is a medicament that comprises a population of IFNγ⁺IL-10⁺IL-34⁺ secreting human CD8⁺CD45RC^{low} Treg cells according to the invention, a medicament that comprises an enriched population of IFNγ⁺IL-10⁺IL-34⁺ secreting human CD8⁺CD45RC^{low} Treg cells according to the invention, a medicament that comprises a population of genetically modified IFNγ⁺IL-10⁺IL-34⁺ secreting human CD8⁺CD45RC^{low} Treg cells according to the invention, a medicament that comprises an expanded population of IFNγ⁺IL-10⁺IL-34⁺ secreting human CD8⁺CD45RC^{low} Treg cells according to the invention, and a medicament that comprises an expanded population of antigen-specific IFNγ⁺IL-10⁺IL-34⁺ secreting human CD8⁺CD45RC^{low} Treg cells according to the invention.

### Therapeutic methods and uses of Treg cells of the invention:

As above-mentioned, an isolated population of IFNγ⁺IL-10⁺IL-34⁺ secreting human CD8⁺CD45RC^{low} Treg cells of the invention is of interest in the fields of autoimmunity, chronic inflammation, allergy, transplantation (either for preventing or treating graft rejection or GVHD), gene therapy and treatment with therapeutic protein, to avoid degradation of self-tissues or therapeutic proteins by the immune system. An isolated population of human CD8⁺CD45RC^{low} Treg cells according to the invention indeed exhibits the ability to induce immune tolerance and/or to suppress and/or inhibit immune responses, preferably antigen-specific immune response(s) directed against the antigen(s) involved in the disease to be treated, and/or unwanted adaptive immune responses mediated by CD4⁺ T cells, CD8⁺ T cells, preferably immune T-cell tolerance or reduced T-cell activation. Preferably, the isolated population of IFNγ⁺IL-10⁺IL-34⁺ secreting human CD8⁺CD45RC^{low} Treg cells of the invention is isolated from the patient to be treated (and will be re-administered to the patient after *ex vivo* expansion).

As mentioned above, an aspect of the invention thus relates to an isolated population of IFNγ⁺IL-10⁺IL-34⁺ secreting human CD8⁺CD45RC^{low} Treg cells of the invention for use as a medicament. Also described is an isolated population of expanded IFNγ⁺IL-10⁺IL-34⁺ secreting human CD8⁺CD45RC^{low} Treg cells of the invention, preferably antigen-specific, for use as a medicament.

The isolated population of IFNγ⁺IL-10⁺IL-34⁺ secreting human CD8⁺CD45RC^{low} Treg cells according to the invention (preferably an expanded antigen-specific population) may be reintroduced to the patient by a number of approaches. Preferably, they are injected intravenously. In one embodiment, about 1×10⁴ to about 1×10⁸ cells are reintroduced in the patient. In another embodiment, about 1×10⁶ to about 10×10⁶ cells/kg of patient body weight are reintroduced to the patient.

The terms "prevent," "preventing," and "prevention" refer herein to the inhibition of the development or onset of a disorder or the prevention of the recurrence, onset, or development of one or more symptoms of a disorder in a subject resulting from the administration of IFNγ⁺IL-10⁺IL-34⁺ secreting human CD8⁺CD45RC^{low} Treg cells according to the present invention.

As used herein, the term "treatment" refers to therapeutic treatment and prophylactic and preventive measures, wherein the object is to prevent or slow down (lessen, diminish) the targeted pathological disorder or condition. Those in need of treatment include those already with the disorder as well as those prone to have the disorder. A patient is successfully "treated" for an unwanted immune response if, after receiving a therapeutic amount of IFNγ⁺IL-10⁺IL-34⁺ secreting human CD8⁺CD45RC^{low} Treg cells according to the present invention, the patient shows observable and/or measurable reduction in or absence of one or more of the following: reduction in the number of pathogenic cells; reduction in the percent of total cells that are pathogenic; and/or relief to some extent, of one or more of the symptoms associated with the specific disease or condition; reduced morbidity and mortality, and improvement in quality of life issues. The above parameters for assessing successful treatment and improvement in the disease are readily measurable by routine procedures familiar to a physician.

As used herein, the term "patient" refers to a human, who is awaiting the receipt of, or is receiving, medical care or was/is/will be the subject of a medical procedure, or is monitored for the development or progression of a disease. In one embodiment, the patient is an adult (for example a patient above the age of 18). In another embodiment, the patient is a child (for example a patient below the age of 18). In one embodiment, the patient is a male. In another embodiment, the patient is a female.

As used herein, the term "therapeutically effective amount" refers to the number of IFNγ⁺IL-10⁺IL-34⁺ secreting human CD8⁺CD45RC^{low} Treg cells that is aimed at, without causing significant negative or adverse side effects to the target, (1) delaying or preventing the onset of an unwanted immune response; (2) slowing down or stopping the progression, aggravation, or deterioration of one or more symptoms of an unwanted immune response; (3) bringing about ameliorations of the symptoms of an unwanted immune response; (4) reducing the severity or incidence of an unwanted immune response; or (5) curing an unwanted immune response. A therapeutically effective amount may be administered prior to the onset of an unwanted immune response, for a prophylactic or preventive action. Alternatively, or additionally, the therapeutically effective amount may be administered after initiation of an unwanted immune response, for a therapeutic action. The effective amount will vary with the age, gender, race, general condition, etc., of the patient, the severity of the condition being treated, the duration of the treatment, the nature of any concurrent treatment, the pharmaceutically acceptable carrier used, and like factors within the knowledge and expertise of those skilled in the art. As appropriate, an "effective amount" in any individual case can be determined by one of ordinary skill in the art by reference to the pertinent texts and literature and/or by using routine experimentation, (for example, see Gennaro et al., Eds. Remington's The Science and Practice of Pharmacy, 20th edition, (2000), Lippincott Williams and Wilkins, Baltimore MD; Braunwald et al., Eds. Harrison's Principles of Internal Medicine, 15th edition, (2001), McGraw Hill, NY; Berkow et al., Eds. The Merck Manual of Diagnosis and Therapy, (1992), Merck Research Laboratories, Rahway NJ).

In one embodiment, the invention relates to an isolated population of IFNγ⁺IL-10⁺IL-34⁺ secreting human CD8⁺CD45RC^{low} Treg cells of the invention for use in a method for preventing or treating an autoimmune disease.

Also described is an isolated population of expanded IFNγ⁺IL-10⁺IL-34⁺ secreting human CD8⁺CD45RC^{low} Treg cells of the invention for use in a method for preventing or treating an autoimmune disease.

Also described is a method for preventing or treating an autoimmune disease comprising the step of administering a pharmaceutically effective amount of a population of IFNγ⁺IL-10⁺IL-34⁺ secreting human CD8⁺CD45RC^{low} Treg cells of the invention to a patient in need thereof.

As used herein, the term "autoimmune disease" refers to a disease in which the immune system produces an immune response (for example, a B-cell or a T-cell response) against an antigen that is part of the normal host (that is an auto-antigen), with consequent injury to tissues. In an autoimmune disease, the immune system of the host fails to recognize a particular antigen as "self" and an immune reaction is mounted against the host's tissues expressing the antigen.

Exemplary autoimmune diseases affecting humans include rheumatoid arthritis, juvenile oligoarthritis, collagen-induced arthritis, adjuvant-induced arthritis, uveitis, Sjogren's syndrome, multiple sclerosis, experimental autoimmune encephalomyelitis, inflammatory bowel disease (for example, Crohn's disease and ulcerative colitis), autoimmune gastric atrophy, pemphigus vulgaris, psoriasis, vitiligo, type 1 diabetes, non-obese diabetes, myasthenia gravis, Grave's disease, Hashimoto's thyroiditis, sclerosing cholangitis, sclerosing sialadenitis, systemic lupus erythematosis, autoimmune thrombocytopenia purpura, Goodpasture's syndrome, Addison's disease, systemic sclerosis, polymyositis, dermatomyositis, acquired hemophilia, thrombotic thrombocytopenic purpura and the like.

In another embodiment, the invention relates to an isolated population of IFNγ⁺IL-10⁺IL-34⁺ secreting human CD8⁺CD45RC^{low} Treg cells of the invention for use in a method for preventing or treating chronic inflammatory diseases.

Also described is an isolated population of expanded IFNγ⁺IL-10⁺IL-34⁺ secreting human CD8⁺CD45RC^{low} Treg cells of the invention for use in a method for preventing or treating chronic inflammatory diseases.

Examples of chronic inflammatory diseases affecting humans include, without being limited to, non-autoimmune inflammatory bowel disease, post-surgical adhesions, coronary artery disease, hepatic fibrosis, acute respiratory distress syndrome, acute inflammatory pancreatitis, endoscopic retrograde cholangiopancreatography-induced pancreatitis, burns, atherogenesis of coronary, cerebral and peripheral arteries, appendicitis, cholecystitis, diverticulitis, visceral fibrotic disorders, wound healing, skin scarring disorders (keloids, hidradenitis suppurativa), granulomatous disorders (sarcoidosis, primary biliary cirrhosis), asthma, pyoderma gandrenosum, Sweet's syndrome, Behcet's disease, primary sclerosing cholangitis, and an abscess
In another embodiment, the invention relates to an isolated population of IFNγ⁺IL-10⁺IL-34⁺ secreting human CD8⁺CD45RC^{low} Treg cells of the invention for use in a method for preventing or treating graft rejection (or for inducing transplant tolerance) or for preventing or treating GVHD

Also described is an isolated population of expanded IFNγ⁺IL-10⁺IL-34⁺ secreting human CD8⁺CD45RC^{low} Treg cells of the invention for use in a method for preventing or treating graft rejection (or for inducing transplant tolerance) or for preventing or treating GVHD

Also described is a method for preventing or treating graft versus host disease (GVHD) or graft rejection (or inducing transplant tolerance) comprising the step of administering a pharmaceutically effective amount of a population of IFNγ⁺IL-10⁺IL-34⁺ secreting human CD8⁺CD45RC^{low} Treg cells of the invention to a patient in need thereof.

As used herein, the term "transplant rejection" encompasses both acute and chronic transplant rejection. "Acute rejection" is the rejection by the immune system of a tissue transplant recipient when the transplanted tissue is immunologically foreign. Acute rejection is characterized by infiltration of the transplant tissue by immune cells of the recipient, which carry out their effector function and destroy the transplant tissue. The onset of acute rejection is rapid and generally occurs in humans within a few weeks after transplant surgery. Generally, acute rejection can be inhibited or suppressed with immunosuppressive drugs such as rapamycin, cyclosporine, anti-CD40L monoclonal antibody and the like. "Chronic transplant rejection" generally occurs in humans within several months to years after engraftment, even in the presence of successful immunosuppression of acute rejection. Fibrosis is a common factor in chronic rejection of all types of organ transplants.

The term "transplantation" and variations thereof refers to the insertion of a transplant (also called graft) into a recipient, whether the transplantation is syngeneic (where the donor and recipient are genetically identical), allogeneic (where the donor and recipient are of different genetic origins but of the same species), or xenogeneic (where the donor and recipient are from different species). Thus, in a typical scenario, the host is human and the graft is an isograft, derived from a human of the same or different genetic origins. In another scenario, the graft is derived from a species different from that into which it is transplanted, including animals from phylogenically widely separated species, for example, a baboon heart being transplanted into a human host.

In one embodiment, the donor of the transplant is a human. The donor of the transplant can be a living donor or a deceased donor, namely a cadaveric donor.

In one embodiment, the transplant is an organ, a tissue, or cells.

As used herein, the term "organ" refers to a solid vascularized organ that performs a specific function or group of functions within an organism. The term organ includes, but is not limited to, heart, lung, kidney, liver, pancreas, skin, uterus, bone, cartilage, small or large bowel, bladder, brain, breast, blood vessels, esophagus, fallopian tube, gallbladder, ovaries, pancreas, prostate, placenta, spinal cord, limb including upper and lower, spleen, stomach, testes, thymus, thyroid, trachea, ureter, urethra. As used herein, the term "tissue" refers to any type of tissue in human or animals, and includes, but is not limited to, vascular tissue, skin tissue, hepatic tissue, pancreatic tissue, neural tissue, urogenital tissue, gastrointestinal tissue, skeletal tissue including bone and cartilage, adipose tissue, connective tissue including tendons and ligaments, amniotic tissue, chorionic tissue, dura, pericardia, muscle tissue, glandular tissue, facial tissue, ophthalmic tissue.

As used herein, the term "cells" refers to a composition enriched for cells of interest, preferably a composition comprising at least about 20%, about 30%, about 40%, preferably at least about 50%, about 60%, even more preferably at least about 65%, about 70%, about 75%, about 80% of said cells.

In certain embodiments the cells are selected from the group consisting of multipotent hematopoietic stem cells derived from bone marrow, peripheral blood, or umbilical cord blood; or pluripotent (i.e. embryonic stem cells (ES) or induced pluripotent stem cells (iPS)) or multipotent stem cell-derived differentiated cells of different cell lineages such as cardiomyocytes, beta-pancreatic cells, hepatocytes, neurons, etc...

In one embodiment, the cell composition is used for allogeneic hematopoietic stem cell transplantation (HSCT) and thus comprises multipotent hematopoietic stem cells, usually derived from bone marrow, peripheral blood, or umbilical cord blood.

HSCT can be curative for patients with leukemia and lymphomas. However, an important limitation of allogeneic HCT is the development of graft versus host disease (GVHD), which occurs in a severe form in about 30-50% of humans who receive this therapy.

In another embodiment, the invention relates to an isolated population of IFNγ⁺IL-10⁺IL-34⁺ secreting human CD8⁺CD45RC^{low} Treg cells of the invention for use in a method for preventing or treating any unwanted immune reaction against a therapeutic protein.

Also described is an isolated population of expanded IFNγ⁺IL-10⁺IL-34⁺ secreting human CD8⁺CD45RC^{low} Treg cells of the invention for use in a method for preventing or treating any unwanted immune reaction against a therapeutic protein.

Also described is a method for preventing or treating any unwanted immune reaction against a therapeutic protein comprising the step of administering a pharmaceutically effective amount of a population of IFNγ⁺IL-10⁺IL-34⁺ secreting human CD8⁺CD45RC^{low} Treg cells of the invention to a patient in need thereof.

As used herein, the term "unwanted immune response against a therapeutic protein" refers to any unwanted immune reaction directed to proteins expressed in the course of gene therapy, and/or therapeutic proteins, such as factor VIII (hemophilia A) and other coagulation factors, enzyme replacement therapies, monoclonal antibodies (e.g. natalizumab, rituximab, infliximab), polyclonal antibodies, enzymes or cytokines (e.g. IFNβ).

In another embodiment, the invention relates to an isolated population of IFNγ⁺IL-10⁺IL-34⁺ secreting human CD8⁺CD45RC^{low} Treg cells of the invention for use in the prevention or the treatment of allergies.

Also described is an isolated population of expanded IFNγ⁺IL-10⁺IL-34⁺ secreting human CD8⁺CD45RC^{low} Treg cells of the invention for use in the prevention or the treatment of allergies.

Also described is a method for preventing or treating allergy comprising the step of administering a pharmaceutically effective amount of a population of IFNγ⁺IL-10⁺IL-34⁺ secreting human CD8⁺CD45RC^{low} Treg cells of the invention to a patient in need thereof.

As used herein, the term "allergy" or "allergies" refers to a disorder (or improper reaction) of the immune system. Allergic reactions occur to normally harmless environmental substances known as allergens; these reactions are acquired, predictable, and rapid. Strictly, allergy is one of four forms of hypersensitivity and is called type I (or immediate) hypersensitivity. It is characterized by excessive activation of certain white blood cells called mast cells and basophils by a type of antibody known as IgE, resulting in an extreme inflammatory response. Common allergic reactions include eczema, hives, hay fever, asthma, food allergies, and reactions to the venom of stinging insects such as wasps and bees.

In one embodiment, the subject is a patient.

In one embodiment, the subject is affected, preferably is diagnosed, with a chronic inflammatory disease

In one embodiment, the subject is affected, preferably is diagnosed, with an autoimmune disease.

In one embodiment, the subject was previously transplanted, or will be transplanted.

In one embodiment, the subject previously received a therapeutic protein, or will receive a therapeutic protein.

In one embodiment, the subject is affected, preferably is diagnosed with allergy.

In one embodiment, the subject is treated with an immunosuppressive drug, such as, for example, rapamycin, cyclosporine A, mycophenolate mofetil, methylprednisolone, tacrolimus or combinations thereof. In another embodiment, the subject is not treated with an immunosuppressive drug, such as, for example, rapamycin, cyclosporine A, mycophenolate mofetil, methylprednisolone, tacrolimus or combinations thereof.

### Prognostic methods of the invention:

In another aspect, the invention relates to an *in vitro* method for determining whether a patient is at risk of transplant rejection, GVHD, chronic inflammatory diseases, autoimmune diseases, unwanted immune response against therapeutic proteins or allergies (as above-defined), comprising a step of determining the presence of IFNγ⁺IL-10⁺IL-34⁺ secreting human CD8⁺CD45RC^{low} Treg cells as described hereinabove in a biological sample obtained from said patient by a method of the invention, wherein the presence of IFNγ⁺IL-10⁺IL-34⁺ secreting human CD8⁺CD45RC^{low} Treg cells as described hereinabove is indicative of a reduced risk of transplant rejection, GVHD, chronic inflammatory diseases, autoimmune diseases, unwanted immune response against therapeutic proteins or allergies.

As used herein, the term "risk" refers to the probability that an event will occur over a specific time period, such as the onset of transplant rejection, and can mean a subject's "absolute" risk or "relative" risk. Absolute risk can be measured with reference to either actual observation post-measurement for the relevant time cohort, or with reference to index values developed from statistically valid historical cohorts that have been followed for the relevant time period. Relative risk refers to the ratio of absolute risks of a patient compared either to the absolute risks of low risk cohorts or an average population risk, which can vary by how clinical risk factors are assessed. Odds ratios, the proportion of positive events to negative events for a given test result, are also commonly used (odds are according to the formula p/(l-p) where p is the probability of event and (1- p) is the probability of no event).

"Risk determination" in the context of the invention encompasses making a prediction of the probability, odds, or likelihood that an event may occur. Risk determination can also comprise prediction of future clinical parameters, traditional laboratory risk factor values, such as age, sex mismatch, HLA-testing, etc ...; either in absolute or relative terms in reference to a previously measured population. The methods of the invention may be used to make categorical measurements of the risk of transplant rejection, thus defining the risk spectrum of a category of transplanted patient defined as being at risk of transplant rejection.

As used herein, the term "determining the presence" includes qualitative and/or quantitative detection (i.e. detecting and/or measuring the presence) with or without reference to a control or a predetermined value. As used herein, "detecting" means determining if IFNγ⁺IL-10⁺IL-34⁺ secreting human CD8⁺CD45RC^{low} Treg cells as described hereinabove are present or not in a biological sample and "measuring" means determining the amount of IFNγ⁺IL-10⁺IL-34⁺ secreting human CD8⁺CD45RC^{low} Treg cells as described hereinabove in a biological sample.

As used herein, the term "biological sample" has its general meaning in the art and refers to any sample which may be obtained from a patient for the purpose of *in vitro* evaluation. A preferred biological sample is a blood sample (e.g. whole blood sample, serum sample, or plasma sample), more particularly a blood sample obtained from a transplanted patient or a patient suffering from an autoimmune disease or allergy.

Also described is an *in vitro* method for determining whether a transplanted patient (recipient) is tolerant, comprising a step of determining the presence of IFNγ⁺IL-10⁺IL-34⁺ secreting human CD8⁺CD45RC^{low} Treg cells as described hereinabove in a biological sample obtained from said transplanted patient by a method of the invention, wherein the presence of IFNγ⁺IL-10⁺IL-34⁺ secreting human CD8⁺CD45RC^{low} Treg cells as described hereinabove is indicative of tolerance.

As used herein, the terms "tolerance" or "immune tolerance" refers to a state of unresponsiveness of the immune system to substances or tissues that have the capacity to elicit an immune response. As used herein, the term "immune response" includes T cell mediated and/or B cell mediated immune responses. Exemplary immune responses include T cell responses, e.g., cytokine production and cellular cytotoxicity, in addition, the term immune response includes immune responses that are indirectly effected by T cell activation, e.g., antibody production (humoral responses) and activation of cytokine responsive cells, e.g., macrophages. Immune cells involved in the immune response include lymphocytes, such as B cells and T cells (CD4⁺, CD8⁺, Th1 and Th2 cells); antigen presenting cells (e.g. professional antigen presenting cells such as dendritic cells); natural killer cells; myeloid cells, such as macrophages, eosinophils, mast cells, basophils, and granulocytes.

The invention will be further illustrated by the following figures and examples. However, these examples and figures should not be interpreted in any way as limiting the scope of the present invention.

### FIGURES:

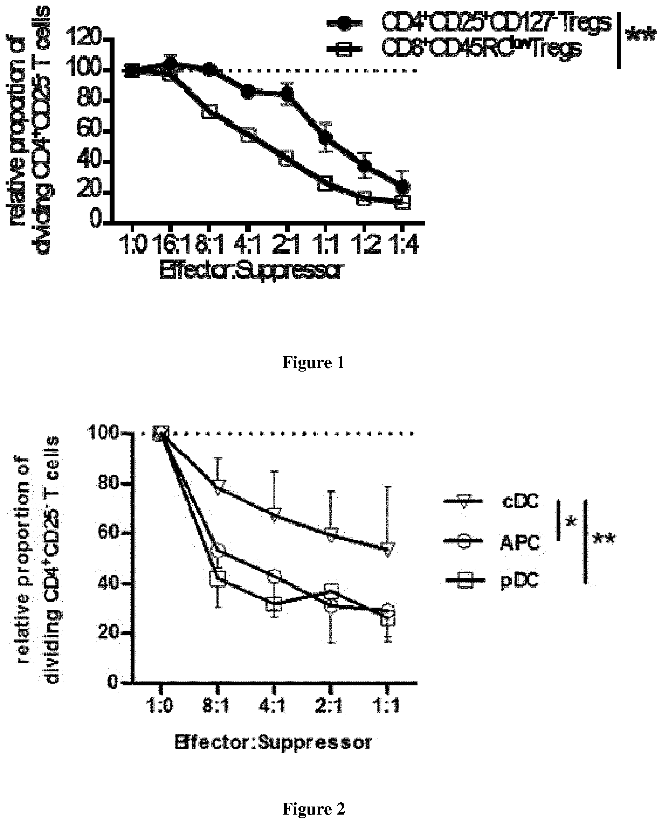

**Figure 1****. Human CD8⁺CD45RC^{low} Tregs suppress more efficiently anti-donor immune responses than human CD4⁺CD25⁺CD127⁻ Tregs.** CD8⁺CD45RC^{low} T cells were compared to classical CD4⁺CD25⁺CD127⁻ Tregs for suppressive activity on proliferation of syngeneic effector CD4⁺CD25⁻ T cells stimulated with allogeneic T-depleted PBMCs, in a range from 16:1 to 1:4 effector:suppressor ratio. Two Way RM ANOVA. Proliferation was normalized to proliferation in absence of Tregs. *, p<0.05.
**Figure 2****. The suppressive activity of human CD8⁺CD45RC^{low} Tregs is preferentially increased by interaction with pDCs.** Sorted CD8+ Tregs were stimulated 12h with anti-CD3 and anti CD28 mAbs and analyzed for suppressive activity on proliferation of syngeneic effector CD4⁺CD25⁻ T cells stimulated with allogeneic T-depleted PBMCs (APC), cDCs or pDCs, in a range from 8:1 to 1:1 effector:suppressor ratio. Two Way RM ANOVA. Proliferation was normalized to proliferation in absence of Tregs. *p<0.05, **, p<0.01.

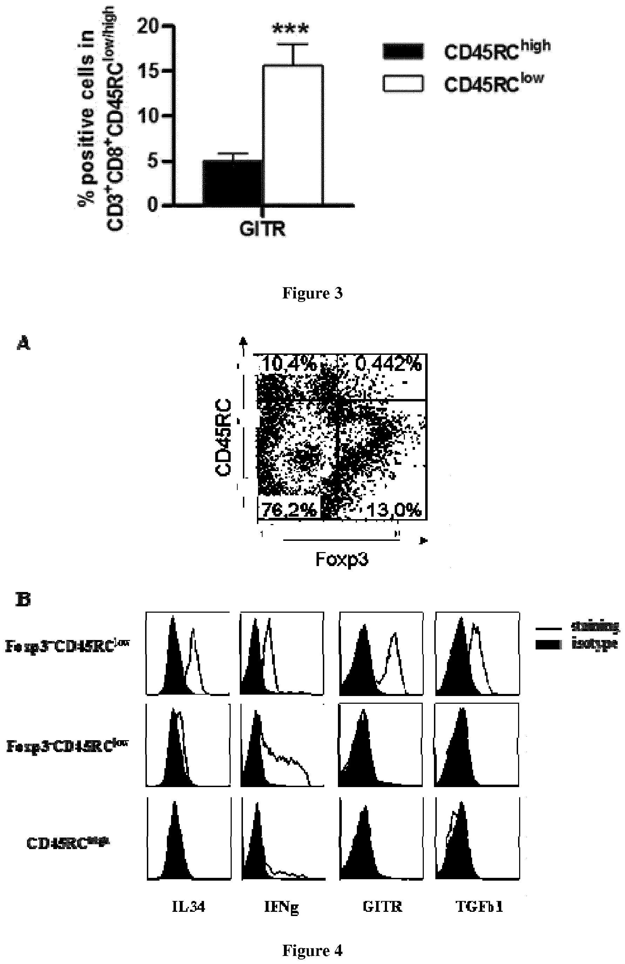

**Figure 3****. A subpopulation of human CD8⁺CD45RC^{low} Tregs expresses GITR.** The expression of the surface receptor GITR was analyzed by flow cytometry in isolated CD8⁺CD45RC^{low} Tregs and isolated CD8⁺CD45RC^{high} Tregs. Results are expressed as the mean percentage of cells (+/- SEM) expressing GITR.
**Figure 4****. Expression of TGFβ-1, IL34, IFNγ and GITR is enriched in Foxp3⁺ human CD8⁺ Tregs.** PBMCs were isolated from the blood of healthy volunteers by Ficoll gradient, and were stimulated for 4h with 50ng/ml PMA and 1µg/ml ionomycine in presence of 10µg/ml Brefeldine A. Isolated CD8⁺ cell populations were analyzed by FACS staining for expression of Foxp3, IL-34, IFNγ, TGFβ-1 and GITR after gating on viable cells. One representative experiment. **A.** Expression of Foxp3 and CD45RC was analyzed in CD8⁺ Tregs. **B.** Expression of IL-34, IFNγ, GITR and TGFβ-1 was determined in Foxp3⁺CD45RC^{low}, Foxp3⁻CD45RC^{low} and CD45RC^{high} CD8⁺ cells.

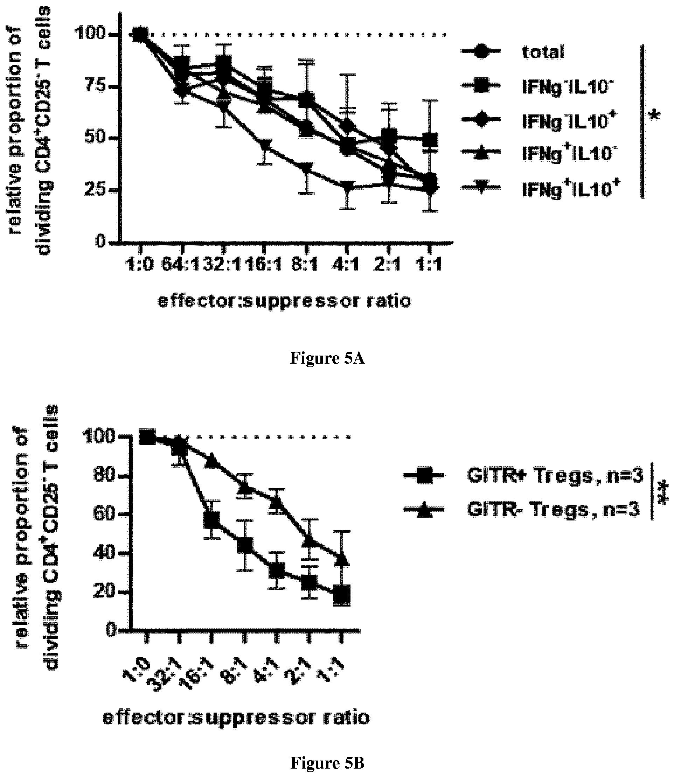

**Figure 5****. Specific subpopulations of human CD8⁺CD45RC^{low} Tregs demonstrate high suppressive capacity *in vitro.*** A. Sorted CD8⁺ Tregs were stimulated 24h with anti-CD3 and anti-CD28 mAbs, sorted on IFNγ and/or IL-10 secretion, and tested for suppressive activity in a range of effector: suppressor ratios in a MLR assay where syngeneic CFSE-labeled CD4⁺CD25⁻ T cells were stimulated by allogeneic T-depleted PBMCs. The sorted CD8⁺ Tregs were compared to total anti-CD3+anti-CD28 mAbs stimulated CD8⁺ Tregs. Two Way ANOVA RM. Proliferation was normalized to proliferation in absence of Tregs. **, p<0.01. **B.** Sorted CD8⁺CD45RC^{low} Tregs were stimulated 12h with 1µg/ml anti-CD3 and anti-CD28 mAbs and newly sorted on GITR expression by FACS Aria. GITR⁺CD8⁺CD45RC^{low} Tregs and GITR⁻CD8⁺CD45RC^{low} Tregs were analyzed for suppressive activity on proliferation of syngeneic effector CD4⁺CD25⁻ T cells stimulated with allogeneic T-depleted PBMCs, in a range from 32:1 to 1:1 effector:suppressor ratio. Two Way RM ANOVA. Proliferation was normalized to proliferation in absence of Tregs. **, p<0.01

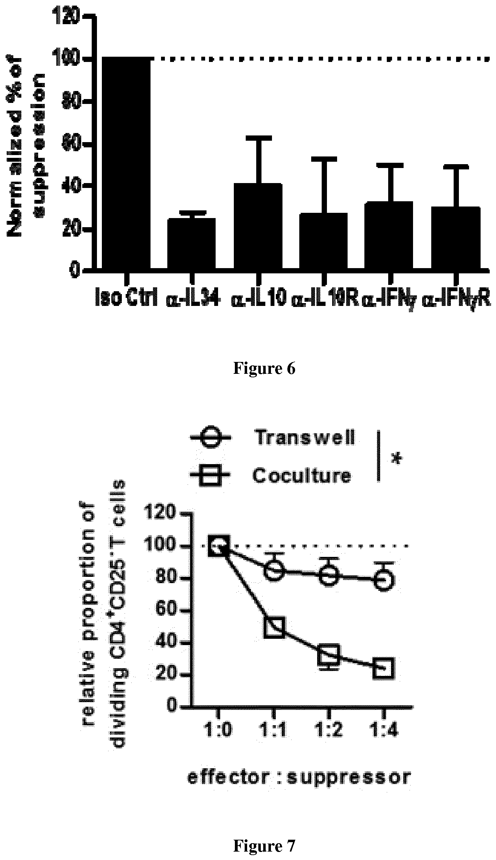

**Figure 6****. IFNγ, IL-10 and IL-34 are key mediators of CD8⁺CD45RC^{low} Tregs suppression.** 50µg/ml of anti-IL-34 (α-IL-34), anti-IL-10 (α-IL-10) or anti-IFNy cytokines (α-IFNγ) or their anti-receptor anti-IL-10R (α-IL-10R) or anti-IFNγR (α -IFNγR) purified blocking Abs were added at day 0 of co-culture assay using sorted CD8⁺CD45RC^{low} T cells, and syngeneic CFSE-labeled effector CD4⁺CD25⁻ T cells stimulated with allogeneic T-depleted PBMCs. Proliferation in presence of Tregs was normalized to proliferation in absence of Tregs. Suppression in presence of blocking Abs was normalized to suppression in presence of isotypic control Ab.
**Figure 7****. The suppressive activity of human CD8⁺CD45RC^{low} Tregs is contact dependent.** Transwell experiments were performed as follow: sorted CD8⁺ Tregs were stimulated 12h with anti-CD3 and anti-CD28 mAbs and analyzed for suppressive activity on proliferation of syngeneic effector CD4⁺CD25⁻ T cells stimulated with allogeneic T-depleted PBMCs following 5-day culture in presence of IL-2. Allogeneic T-depleted PBMCs were added in both the upper and the bottom well. The Tregs were added in the upper well and the CD4⁺CD25⁻ T cells in the bottom well, in a range from 1:1 to 1:4 effector:suppressor ratio. Two Way RM ANOVA. Proliferation was normalized to proliferation in absence of Tregs. *, p<0.05.

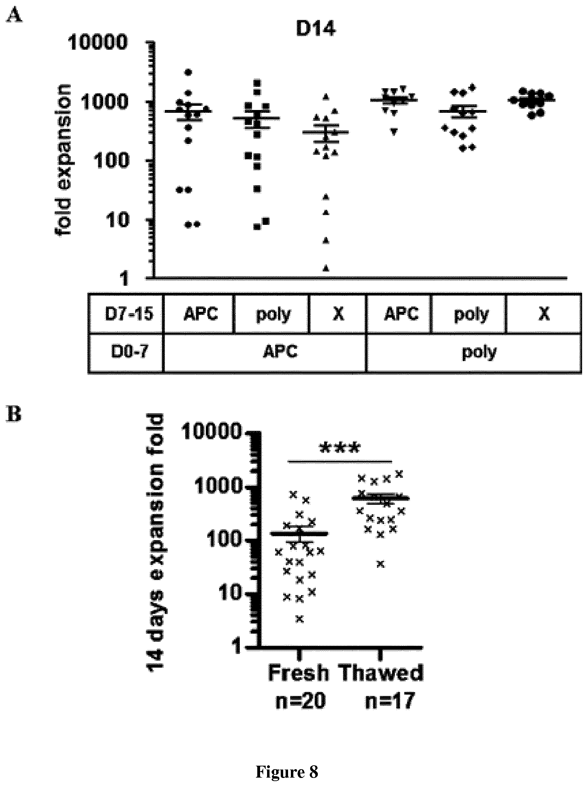

**Figure 8****. Significant expansion of both freshly sorted and thawed CD8⁺CD45RC^{low} Tregs following 14-day stimulation. A.** Sorted CD8⁺ Tregs were stimulated at day 0 with allogeneic APCs (APC) optionally with anti-CD3 and anti-CD28 mAbs (poly), and stimulated again or not (noted X) with the same APCs (APC) or APCs and anti-CD3+anti-CD28 mAbs (poly). The graph represents fold expansion of cells obtained in 14-day culture. **B.** Freshly sorted (fresh) or thawed CD8⁺CD45RC^{low} cells were seeded at 3.3x10⁵ cells/ml in complete medium with allogeneic T-depleted PBMCs at 1:4 Tregs:APCs ratio and 1µg/ml anti-CD3 and anti-CD28 Abs at day 0. On day 7, the Tregs were stimulated again with 1µg/ml anti-CD3 and anti-CD28. On days 0, 7, 10, and 12, the culture medium was supplemented with 1000U/ml human IL-2 and 10ng/ml human IL-15. On day 14, the Tregs were analyzed for expansion yield. The graph represents fold expansion of cells obtained in 14-day culture. Thawed cells show significantly increased expansion. Mann Whitney t test ***, p<0.001.

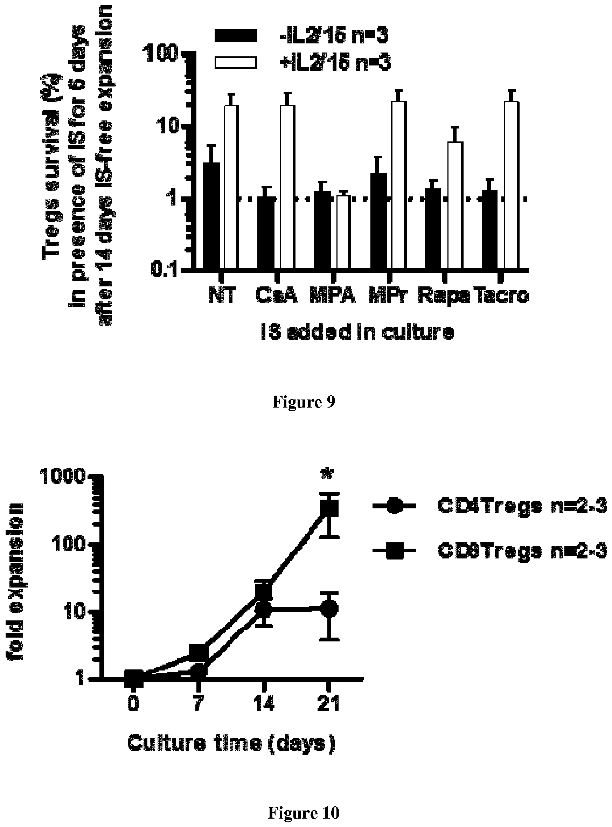

**Figure 9****. Expanded CD8+ Tregs survive in presence of immunosuppressive drugs.** Sorted CD8+ Tregs were stimulated at day 0 with allogeneic T-depleted PBMCs at 1:4 Tregs:APCs ratio and 1µg/ml anti-CD3 and anti-CD28 Abs. On day 7, Tregs were stimulated again with 1µg/ml anti-CD3 and anti-CD28. On days 0, 7, 10 and 12, the culture medium was supplemented with human IL-2 (1000U/ml) and human IL-15 (10ng/ml). On day 14, Tregs were stimulated again with allogeneic APCs, in presence or not (Non Treated - NT) of immunosuppressive drugs (IS): cyclosporine A (CsA), mycophenolate mofetil (MPA), methylprednisolone (MPr), rapamycin (Rapa), or tacrolimus (Tacro) and in presence or not of IL-2/IL-15. Treg survival was analyzed 6 days later. The graph represents the percentage of cell survival obtained following the last 6 days of culture. Percent of survival was normalized to proliferation at day 14 before addition of immunosuppressive drugs (IS).
**Figure 10****. CD8⁺ Tregs proliferate more efficiently than CD4⁺ Tregs in the same culture conditions.** Sorted CD8⁺CD45RC^{low} (CD8⁺) and CD4⁺CD25^{high}CD127^{low} (CD4⁺) Tregs were stimulated with allogeneic T-depleted PBMCs at 1:4 Tregs:APCs ratio and 1µg/ml anti-CD3 and anti-CD28 Abs at day 0. On days 7 and 14, Tregs were stimulated again with 1µg/ml anti-CD3 and anti-CD28. On days 0, 7, 10, 12, 14, 17, and 19 the culture medium was supplemented with 1000U/ml human IL-2 and 10ng/ml human IL-15. Treg expansion yield was analyzed every 7 days. Two-way RM Bonferroni post test *, p<0.05

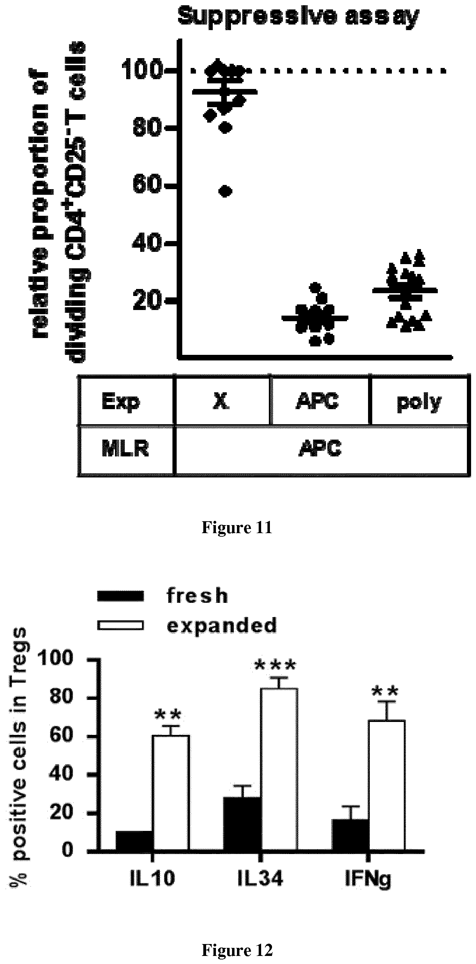

**Figure 11****. Expanded CD8⁺CD45RC^{low} Tregs are significantly more efficient at suppressing allogeneic CD4⁺CD25⁻ effector T cell responses.** Tregs expanded for 7 days with APCs or anti-CD3 + anti-CD28 mAbs + APCs were tested for suppressive activity on syngeneic CD4⁺CD25⁻ T cells (sorted from the donor of Tregs) stimulated with the allogeneic APCs used for expansion. Thawed, sorted and non-expanded Tregs (noted X) from the same donor defined the initial suppressive activity. Proliferation was normalized to proliferation in absence of Tregs.
**Figure 12****. Expanded CD8⁺CD45RC^{low} Tregs are enriched in IFNγ, IL-10 and IL-34-secreting Tregs.** The cytokine expression (IL-10, IL-34 and IFNγ) of 14-day expanded CD8⁺CD45RC^{low} Tregs ("expanded") was analyzed and compared to that of CD8⁺CD45RC^{low} Tregs before expansion ("fresh"). **p<0.01, ***p<0.001.

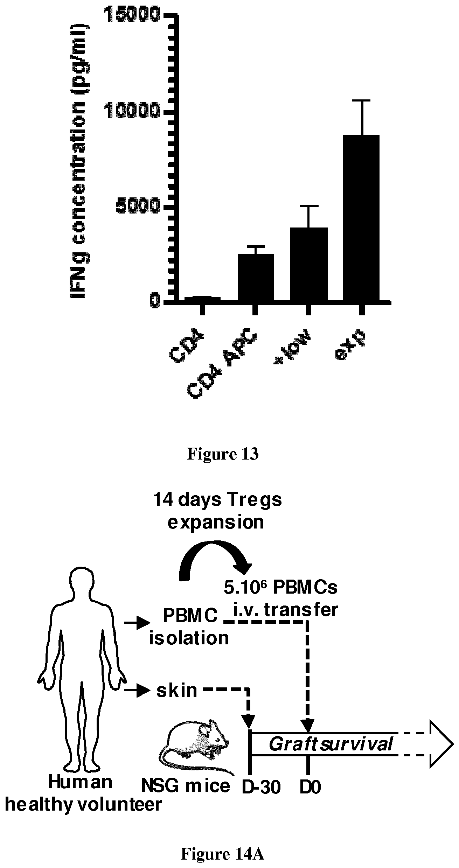

**Figure 13****. Increased secretion of IFNγ can be detected in the supernatant of CD8⁺CD45RC^{low} Tregs.** CD8⁺CD45RC^{low} cells were stimulated with 1µg/ml anti-CD3 and anti-CD28 Abs overnight and either added in a MLR assay where syngeneic effector CD4+CD25-T cells were stimulated with allogeneic T-depleted PBMCs in a 1:1 effector:suppressor ratio, or seeded at 3,3x10⁵ cells/ml in complete medium with allogeneic T-depleted PBMCs at 1:4 Tregs:APCs ratio and 1µg/ml anti-CD3 and anti-CD28 Abs for 14-day expansion. On day 7, the Tregs were stimulated again with 1µg/ml anti-CD3 and anti-CD28 Abs and the medium culture was changed. On days 0, 7, 10, and 12, the culture medium was supplemented with 1000U/ml human IL-2 and 10ng/ml human IL-15. IFNγ was measured in culture supernatants by ELISA on day 14.

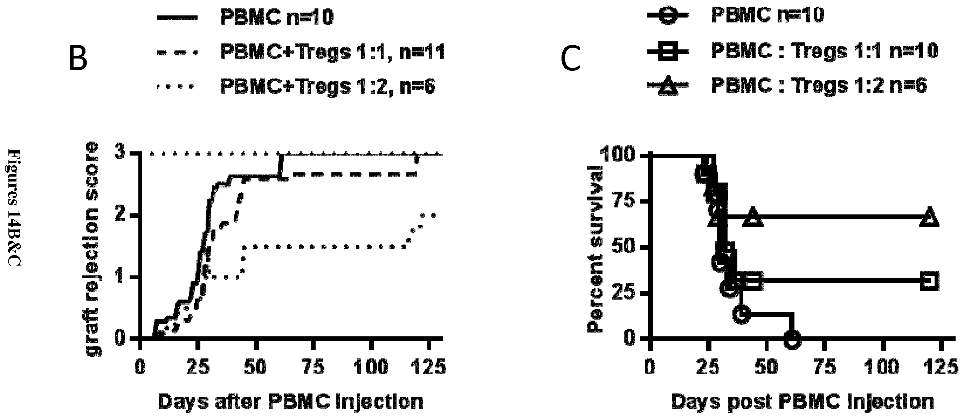

**Figure 14****. Expanded CD8⁺CD45RC^{low} Tregs efficiently suppress allogeneic human skin rejection. A.** Human skin was grafted on NSG mice and left 15 days for acceptance. 5x10⁶ PBMCs allogeneic to the graft were then transferred intraperitoneally with or without syngeneic 15 days expanded Tregs (eTregs). **B.** Graft rejection was evaluated and scored. C. The percentage of mice survival was assessed.

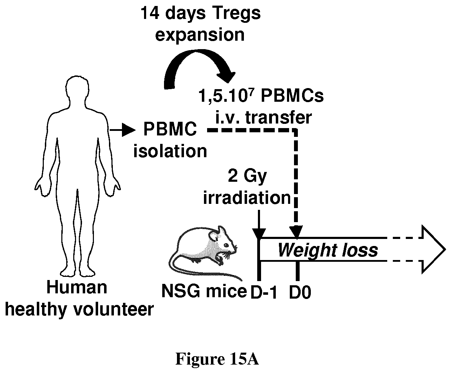

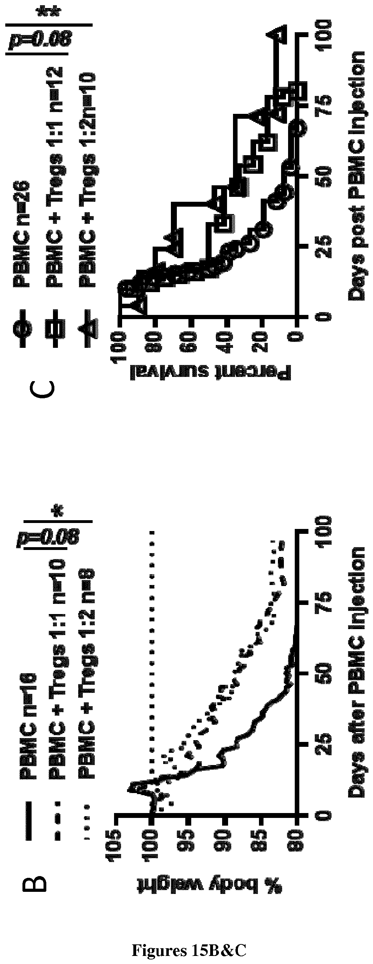

**Figure 15****. Expanded CD8⁺CD45RC^{low} Tregs efficiently suppress GVHD in humanized mice. A.** For xenogeneic GVHD experiments, 1.5x10⁷ syngeneic PBMCs were intravenously transferred with or without polyclonally expanded Tregs (eTregs) at 1:1 and 1:2 PBMCs:expanded Tregs ratio in 12h-previously irradiated NSG mice. **B.** Human PBMCs engraftment was monitored in blood, and GVH development was evaluated by weight loss. **C.** The percentage of mice survival was assessed. *p<0.05, **p<0.01.

### EXAMPLE: A new population of IFNγ⁺IL-10⁺IL-34⁺ secreting human CD8⁺CD45RC^{low} Tregs efficiently suppresses graft rejection.

### Material & Methods

### Healthy volunteers blood collection and PBMC separation:

Blood was collected from healthy donors at the Etablissement Français du Sang (Nantes, France). Approval for this study was obtained from the institutional review boards. Written informed consent was provided according to institutional guidelines. Blood was diluted 2-fold with PBS before PBMC were isolated by Ficoll-Paque density-gradient centrifugation (Eurobio, Courtaboeuf, France) at 2000 rpm for 20 at room temperature without braking. Collected PBMC were washed in 50 mL PBS at 1800 rpm for 10 min and remaining red cells and platelets are eliminated after incubation 5 min in a hypotonic solution and centrifugation at 1000 rpm for 10 min. When indicated, PBMCs were frozen in DMSO:SVF 1:9 and washed twice in medium-10% SVF for thawing.

### Cell isolation:

T cells were obtained from PBMCs by negative selection by elutriation (DTC Plateforme, Nantes) and magnetic depletion (Dynabeads, Invitrogen) of B cells (CD19⁺ cells) and remaining monocytes (CD14⁺ and CD16⁺ cells) before sorting of CD3⁺CD4⁺CD25⁻ cells as responder cells, CD3⁺CD4⁻CD45RC^{low} as CD8⁺ Tregs and CD3⁺CD4⁺CD25⁻CD127^{low} cells as CD4⁺ Tregs cells. Tregs sorted from thawed PBMCs were stimulated 24h with anti-CD3 and anti-CD28 mAbs in presence of 250U/ml IL-2 before plating. IFNγ and/or IL-10 secreting cells were sorted after 24h polyclonal stimulation of Tregs using secretion assay detection kits from Miltenyi. A FACS ARIA I (BD Biosciences, Mountain View, CA) was used to sort cells.

APCs used as stimulator cells were obtained by magnetic depletion of CD3⁺ cells and 35Gy irradiation. pDCs and cDCs were obtained by CD3, CD14, and CD16 positive cells depletion and Nrp1⁺ or CD1c⁺ cell sorting respectively.

### Monoclonal antibodies and flow cytometry:

For interleukins, IFNγ and Foxp3 analysis, PBMCs were stimulated with PMA (50ng/ml) and ionomycine (1µg/ml) for 7h in presence of Brefeldine A (10µg/ml) for the last 4h.

Fluorescence was measured with a LSR II or a Canto II cytometer (BD Biosciences, Mountain View, CA) for phenotype and functional analysis respectively, and the FLOWJO software (Tree Star, Inc. USA) was used to analyze data. Cells were first gated by their morphology excluding dead cells by selecting viable cells.

### Mixed lymphocyte reaction:

Tregs suppressive activity was assessed on syngeneic effector CD4⁺CD25⁻ T cells (obtained from the donor of Tregs) stimulated with allogeneic APCs. A range of stimulator: effector ratio was tested to reach significant proliferation of responder cells when stimulated with DCs, other experiments were realized with 1:1 APCs:responder ratio. Blocking mAbs or isotypic control mAbs were added at 50µg/ml at day 0 of co-culture. Transwell membrane (0.4µM Ø) was used to seclude Tregs from responder cells, both in contact with stimulator cells. 1000U/ml IL-2 (Miltenyi) was added to assess cytokine deprivation by Tregs. Suppressive activity of expanded Tregs from thawed PBMCs was assessed on syngeneic effector cells stimulated with the allogeneic APCs from the same donor that was used for expansion, and compared to unstimulated Tregs sorted from the same donor of thawed PBMCs. Proliferation of CFSE-labeled responder cells was analyzed by flow cytometry after 4.5 days of coculture in 5% AB serum medium, by gating on CD3⁺CD4⁺ living cells (DAPI negative) and excluding of CPD-V450 labeled CD4⁺Tregs when added.

### Expansion of Tregs:

Tregs were seeded at about 3x10⁵/ml in 5% AB serum-medium supplemented with IL-2 (1000U/ml) and IL-15 (10ng/ml) and were stimulated with coated anti-CD3 mAb (1µg/ml), soluble anti-CD28 mAb (1µg/ml) and/or allogeneic APCs at 1:4 Tregs:APCs ratio. At day 7, expanded cells were diluted at 1x10⁵/ml and stimulated again or not. IL-2 and IL-15 cytokines were freshly added at days 0, 7, 10 and 12. At day 14, expanded Tregs were washed with PBS before use.

### Humanized mice models:

8-12 week old NOD/SCID/IL-2Rγ ^{-/-} (NSG) mice were bred in our own animal facilities in SPF conditions. This study was carried out in strict accordance with the protocol approved by the Committee on the Ethics of Animal Experiments of Pays de la Loire (permit number CEEA.2012.155).

For xenogeneic GVHD experiments, 1.5x10⁷ syngeneic PBMCs were intravenously transferred with or without polyclonally expanded Tregs at 1:1 and 1:2 PBMCs:expanded Tregs ratio in 12h-previously irradiated NSG mice. Human PBMCs engraftment was monitored in blood, and GVH development was evaluated by weight loss and histopathological analysis of lung, livers, intestines, spleen, kidneys.

For transplantation model, human skin was provided by CHU Nantes from healthy volunteers and graft was accepted in 15 days by NSG mice. 5x10⁶ PBMCs allogeneic to the graft were transferred intraperitoneally with or without syngeneic 15 days expanded Tregs. Graft rejection score was evaluated.

### Results

### Human CD8⁺CD45RC^{low} Tregs are more efficient suppressors of anti-donor immune responses than human CD4⁺CD25⁺CD127⁻ Tregs.

Previous studies have described the involvement of CD8⁺CD45RC^{low} T regulatory cells in graft acceptance induced by treatment with CD40Ig in a MHC-mismatched heart allograft rat model (Guillonneau *et al.,* 2007; Li *et al.,* 2010). Both studies have underlined the key role of IFNγ in the CD40Ig-induced allograft acceptance.

Phenotypic analysis of the CD45RC marker in healthy individuals showed that the CD45RC marker is differentially represented in healthy individuals as revealed by the overlay of several individuals. This differential expression was not related to age or gender.

First, the suppressive activity of the total human CD8⁺CD45RC^{low} T cell subset was assessed and compared to the suppressive activity of the well-known CD4⁺CD25⁺CD127⁻ Tregs when added at different ratios in a MLR assay where CFSE-labeled CD4⁺CD25⁻ T cells were stimulated by allogeneic T-depleted PBMCs **(****Figure 1****).** Interestingly, human CD8⁺CD45RC^{low} Tregs display a significantly superior suppressive capacity on anti-donor CD4⁺CD25⁻ effector T cells proliferation compared to CD4⁺CD25⁺CD127⁻ Tregs at all ratios.

In the rat, a preferential interaction of CD8⁺CD45RC^{low} Tregs with pDCs was identified for CD8⁺CD45RC^{low} Tregs to exert an optimal suppressive activity. Thus, the suppressive potential of human CD8⁺CD45RC^{low} was tested in presence of different ratios of cDCs (CD3⁻CD19⁻CD1c⁺Nrp-1⁻), pDCs (CD3⁺CD19⁻CD1c⁻Nrp-1⁺) in comparison to T-depleted PBMCs as stimulators **(****Figure 2****).** Interestingly, a lesser suppressive activity of CD8⁺CD45RC^{low} Tregs was observed in presence of cDCs compared to pDCs and T-depleted PBMCs, suggesting that in human, as it was the case in rat, pDCs are necessary to mediate CD8⁺CD45RC^{low} Tregs suppressive activity on CD4⁺ effector T cells.

In conclusion, human CD8⁺CD45RC^{low} Tregs in presence of pDCs suppress more efficiently anti-donor CD4⁺CD25⁺ effector T cells responses than CD4⁺CD25+CD127- Tregs.

### A subpopulation of CD8⁺CD45RC^{low} Tregs expresses IFNγ, IL-34 and IL-10.

To characterize human CD8⁺CD45RC^{low} Treg, a deep phenotypic analysis was performed using 13-colour flow cytometry. Several markers were analyzed to assess the existence of subpopulations within the total CD8⁺CD45RC^{low} Treg subset: markers previously described on Tregs such as CD103, CTLA-4, GITR or cytokines such as IFNγ, IL-34 or IL-10, as well as the known marker of human CD8⁺ Tregs, i.e. CD28 and CD122.

Screening of markers revealed a significant upregulation of CD69, CD71, CD103, Foxp3, Tbet, HLA-DR, CD154, IFNγ, IL-2 and IL-34 expression and a decreased CD38 expression among CD8⁺CD45RC^{low} Tregs compared to CD8⁺CD45RC^{high} T cells, altogether suggesting a profile of activated pro-tolerogenic cells.

Co-expression analysis of CD45RC, CD28 and CD122 revealed that each-subpopulations existed inside the CD45RC marker but that the CD8⁺CD28⁻ Tregs and the CD8⁺CD122⁺ Tregs were not all contained within the CD45RC^{low} marker, suggesting that each of these markers identifies distinct Tregs sub-populations.

Of particular interest, a significant expression of IFNγ and IL-34 was observed among CD8⁺CD45RC^{low} Tregs (about 60% and 20% respectively), with IL-34 being mostly co-expressed by IL-10-secreting cells.

The expression of cell the surface receptor GITR was assessed in CD8⁺CD45RC^{low} Tregs and CD8⁺CD45RC^{high} Tregs **(****Figure 3****).** Flow cytometry analysis showed that GITR is significantly more expressed in the population of CD8⁺CD45RC^{low} Tregs (about 15% of the cells express GITR) than in the population of CD8⁺CD45RC^{high} Tregs (about 5% of the cells express GITR). Tus, among the CD8⁺CD45RC^{low} Tregs there is a subpopulation of GITR⁺ CD8⁺CD45RC^{low} Tregs.

Finally, CD3⁺CD8⁺CD45RC^{low} Tregs were analyzed by FACS staining for expression of Foxp3, IL-34, IFNγ, TGFβ-1 and GITR after gating on viable cells **(****Figure 4****).** The CD3⁺CD8⁺CD45RC^{low} Tregs were obtained from PBMCs stimulated for 4h with 50ng/ml PMA and 1µg/ml ionomycine in presence of 10µg/ml Brefeldine A. Only Foxp3⁺CD8⁺CD45RC^{low} Tregs expressed GITR, TGFb1 and IL-34. Foxp3⁺CD8⁺CD45RC^{low} Tregs also expressed higher levels of IFNγ than Foxp3⁻CD8⁺CD45RC^{low} Tregs.

In conclusion, expression of IL-34, TGFb1 and GITR tightly correlates with Foxp3 expression in CD8⁺CD45RC^{low} Tregs.

### The subpopulation of IL-10⁺IL-34⁺IFNγ⁺-secreting CD8⁺CD45RC^{low} Tregs displays a high suppressive capacity in vitro.

The suppressive potential of the subpopulation of human CD8⁺CD45RC^{low} Tregs expressing IFNγ and IL-34 was assessed. First, subpopulations of CD8⁺CD45RC^{low} Tregs were sorted based on the expression of IFNγ and IL-10 using the IFNγ- or IL-10- secretion assay - detection kit allowing sorting of live cells. From the observation that IL-34 is mostly co-expressed by IL-10-secreting cells, it was assumed that IL-10⁺ cells were also IL-34⁺ cells. Thus, four subpopulations of CD8⁺CD45RC^{low} Tregs were sorted: IL-10⁻(IL-34⁻)IFNγ⁻, IL-10⁻ (IL-34⁻)IFNγ⁺, IL-10⁺(IL-34⁺)IFNγ⁻, and IL-10⁺(IL-34⁺)IFNγ⁺ CD8⁺CD45RC^{low} Tregs. Such sorted cells were then added in the MLR assay as described above **(****Figure 5A****).** Importantly, the IL-10⁺(IL-34⁺)IFNγ⁺ CD8⁺CD45RC^{low} Tregs were significantly more suppressive than the IL-10⁻(IL-34⁻)IFNγ⁺CD8⁺CD45RC^{low} Tregs, suggesting a key role for IL-10 and IL-34.

In conclusion, the subpopulation of IL-10⁺IL-34⁺IFNγ⁺-secreting CD8⁺CD45RC^{low} Tregs displays a high suppressive capacity *in vitro.*

### GITR⁺ sorted CD8⁺CD45RC^{low} Tregs are more efficient suppressor than GITR⁻ CD8⁺CD45RC^{low} Treg

Similar assays were performed by subdividing the total CD8⁺CD45RC^{low} Treg population with cell-surface markers: GITR, CD38, HLA-DR, CD45RA, CD127, CD197, CD27, CD28, CD25. Although a small loss of suppressive activity could be observed with some other markers such as CD45RA and CD28, there were no significant differences. With one exception, there was no increased suppressive activity, suggesting that these markers cannot be used to further discriminate CD8⁺CD45RC^{low} Tregs with suppressive activity.

However, most interestingly, GITR⁺CD3⁺CD8⁺CD45RC^{low} Tregs more efficiently suppressed anti-donor immune responses than GITR⁻CD3⁺CD8⁺CD45RC^{low} Tregs **(****Figure 5B****).** In conclusion, GITR expression identifies a subpopulation of CD8+ Tregs with a higher suppressive capacity on allogeneic effector CD4⁺CD25⁻ T cell proliferation.

### CD8⁺CD45RC^{low} Treg mediated suppressive activity depends on IL-34, IL-10 and IFNγ secretion.

The different mechanisms of action that could underlie the suppressive activity of the CD8⁺CD45RC^{low} Tregs were then reviewed. Such mechanisms of action include cytokine secretion, contact dependent activity, IL-2 deprivation or killing to suppress effector T cell proliferation.

First, since IFNγ, IL-34 and IL-10 discriminate the CD8⁺CD45RC^{low} Treg subpopulation with the best regulatory activity, cytokine production was tested in the MLR supernatant. Analysis of cytokine production in the MLR's supernatant by day 5 demonstrated a significant production of IFNγ in presence of CD8⁺CD45RC^{low} Treg versus no Tregs and CD8⁺CD45RC^{high} T cells **(****Figure 13****).**

To further assess the role of IFNγ, IL-34 and IL-10 in CD8⁺CD45RC^{low} Treg-mediated suppression, the following blocking antibodies were added in a suppressive MLR assay in presence of CD8⁺CD45RC^{low} Tregs: anti-IL-34, anti-IL-10, anti-IL-10R, anti-IFNγ and anti-IFNγR **(****Figure 6****).** Interestingly, blockade of IL-34, IL-10 and IL-10R, IFNγ and IFNγR resulted in a similar loss of suppressive activity of the CD8⁺CD45RC^{low} Tregs on the CD4⁺CD25⁻ T cell anti-donor immune response.

### CD8⁺CD45RC^{low} Treg mediated suppressive activity also depends on contact.

To determine the importance of a contact for CD8⁺CD45RC^{low} Tregs mediated suppression, transwell experiments were performed **(****Figure 7****).** The proliferation of CFSE-labeled CD4⁺CD25⁻ T cells stimulated with allogeneic T-depleted PBMCs in the lower chamber was assessed in presence or not of CD8⁺CD45RC^{low} Tregs in the lower or upper chamber. A complete loss of suppressive activity was observed when CD8⁺CD45RC^{low} Tregs were added separately in the upper chamber, demonstrating the requirement for a contact.

As this could suggest suppressive mechanisms through killing, next increasing ratios of CD8⁺CD45RC^{low} Tregs-mediated cytotoxicity were tested towards syngeneic CD4⁺CD25⁻ effector T cells or allogeneic T-depleted PBMCs following 15h incubation. There was no necrosis or late apoptosis induction toward syngeneic or allogeneic targets, however a small early apoptosis induction was observed toward both targets at high target:effector ratios (data not shown). The absence of end-stage apoptosis induction suggests that cytotoxic mechanisms play little role in the suppressive effect mediated by the CD8⁺CD45RC^{low} Tregs.

### CD8⁺CD45RC^{low} Tregs can be efficiently expanded, even after thawing.

As cell therapy using CD4⁺ Tregs is a promising issue to prevent allograft from rejection, and as CD8⁺CD45RC^{low} Tregs are more efficient than CD4⁺CD25^{high}CD127⁻ Tregs to inhibit anti-donor immune response *ex vivo* **(****Figure 1****),** a protocol of expansion of CD8⁺CD45RC^{low} Tregs was set up.

First the most efficient conditions for expansion of total CD8⁺CD45RC^{low} Tregs were identified using different ratios of APCs (from 1:1 to 1:16 Treg:APCs ratios), different sources of sera (AB serum, autologous plasma, Tex Mecs or albumine 2%) and different stimulations (polyclonal anti-CD3 + anti-CD28 vs pooled allogeneic T-depleted APCs).

It was thus found that a 1:4 Treg:APC ratio was necessary and sufficient for efficient 7-days Tregs expansion and that the use of AB serum in the culture media led to efficient CD8⁺CD45RC^{low} Tregs expansion at such ratio. It was next observed that CD8⁺CD45RC^{low} Tregs can be efficiently expanded until 1000-fold in 14 days, even after thawing, when stimulated indifferently with allogeneic T-depleted APCs with or without polyclonal anti-CD3 + anti-CD28 mAbs from day 0 to day 7 of culture, in presence of high dose of IL-2 and IL-15, and re-stimulated or from day 7 to day 14 **(****Figure 8A****).**

The expansion of freshly sorted CD8⁺CD45RC^{low} Tregs and thawed CD8⁺CD45RC^{low} Tregs was compared after 14 days of culture **(****Figure 8B****).** On day 0, the cells were stimulated with allogeneic T-depleted PBMCs at 1:4 Tregs:APCs ratio and with polyclonal anti-CD3 + anti-CD28 Abs. On day 7, the cells were stimulated again with anti-CD3 + anti-CD28 Abs. On days 0, 7, 10 and 12 the culture medium was supplemented with IL-2 and IL-15. After 14 days, freshly sorted CD8⁺CD45RC^{low} Tregs were expanded 135-fold and thawed CD8⁺CD45RC^{low} Tregs were expanded 612-fold. Thus, thawed CD8⁺CD45RC^{low} Tregs proliferated more efficiently than freshly sorted CD8⁺CD45RC^{low} Tregs.

In conclusion, CD8⁺CD45RC^{low} Tregs can efficiently be expanded, even more so after thawing.

Furthermore, the survival of expanded CD8⁺CD45RC^{low} Tregs in presence of immunosuppressive drugs was assessed **(****Figure 9****)**. On day 0, the cells were stimulated with allogeneic T-depleted PBMCs at 1:4 Tregs:APCs ratio and with polyclonal anti-CD3 + anti-CD28 mAbs. On day 7, the cells were stimulated again with anti-CD3 + anti-CD28 mAbs. On days 0, 7, 10 and 12 the culture medium was supplemented with IL-2 and IL-15. On day 14, the Tregs were stimulated again with allogeneic APCs, in presence or not of immunosuppressive drugs (cyclosporine A, mycophenolate mofetil, methylprednisolone, rapamycin, or tacrolimus) and in presence or not of IL-2/IL-15. On day 20, the Tregs were analyzed for survival. CD8⁺CD45RC^{low} Tregs survival was not altered in presence of immunosuppressive drugs. Furthermore, CD8⁺CD45RC^{low} Tregs proliferated in presence of the immunosuppressive drugs tested, with the exception of mycophenolate mofetil. Proliferation was more efficient with IL-2/IL-15 supplementation, suggesting that both cytokines could be added *in vivo* in supplementation of CD8⁺ Tregs to maintain their proliferation.

Finally, the proliferation of CD8⁺CD45RC^{low} Tregs and CD4⁺CD25^{high}CD127^{low} Tregs was compared in the same culture conditions **(****Figure 10****)**. On day 0, both subpopulations of Tregs were stimulated for 21 days with allogeneic T-depleted PBMCs at 1:4 Tregs:APCs ratio and with polyclonal anti-CD3 + anti-CD28 mAbs. On days 7 and 14, the Tregs were stimulated again with anti-CD3 + anti-CD28 mAbs. On days 0, 7, 10, 12, 14 and 19 the culture medium was supplemented with IL-2 and IL-15. CD8⁺CD45RC^{low} Tregs were expanded 354-fold whereas CD4⁺CD25^{high} Tregs were only expanded 11-fold in response to polyclonal stimulation. Furthermore, efficient and exponential CD8⁺CD45RC^{low} Treg proliferation was maintained until at least day 21 in contrast to CD4⁺CD25^{high} Treg proliferation that reached a plateau on day 14.

### 14-days expanded CDS⁺CD45RC^{low} Tregs upregulates IFNγ, IL-10 and IL-34 to inhibit immune rejection.

Importantly, both allogeneic and polyclonally expanded CD8⁺CD4SRC^{low} Tregs were significantly more efficient at suppressing allogeneic CD4⁺CD25⁻ effector T cell responses than thawed, sorted and non-expandedCD8⁺CD4SRC^{low} Tregs **(****Figure 11****).**

In addition, such 14-day expanded CD8⁺CD4SRC^{low} Tregs displayed comparable cytotoxic activity than non-expanded or 7-day expanded CD8⁺CD4SRC^{low} Tregs, demonstrating that the superior suppressive capacity acquired upon expansion was not due to increased killing activity of the CD8⁺CD4SRC^{low} Tregs.

Most importantly, such protocol of expansion resulted in significant enrichment of IFNγ⁺IL-34⁺IL-10-secreting CD8⁺CD4SRC^{low} Tregs **(****Figure 12****).**

### IPNγ likely plays a crucial role in CD8⁺CD45RC^{low} Treg function

Detection of IPNγ was assessed in the supernatant of different CD8⁺CD4SRC^{low} Treg cultures **(****Figure 13****).** Isolated CD8⁺CD4SRC^{low} Tregs were stimulated with polyclonal anti-CD3 and anti-CD28 Abs overnight and either added in a MLR assay where syngeneic effector CD4⁺CD25⁻T cells were stimulated with allogeneic T-depleted PBMCs in a 1:1 effector:suppressor ratio, or stimulated with allogeneic T-depleted PBMCs at 1:4 Tregs:APCs ratio at day 0 and polyclonal anti-CD3 and anti-CD28 Abs for 14-day expansion. On day 7, cells were stimulated again with anti-CD3 and anti-CD28 Abs. On days 0, 7, 10, and 12, the culture medium was supplemented with IL-2 and IL-15. IPNγ was measured in culture supernatant by ELISA on day 14. IPNγ secretion was detectable in presence of effector CD4⁺CD25⁻ T cells and APCs (mean 2.5ng/ml), but increased following 6-day incubation with CD8⁺ Tregs (mean 3.9ng/ml) or 14-day expansion of CD8+ Tregs (mean 8,7 ng/ml) with anti-CD3 and anti CD28 mAbs.

In conclusion, high levels of IPNγ are detectable in the supernatants of CD8⁺CD4SRC^{low} Treg culture, suggesting a crucial role for this cytokine in CD8⁺CD4SRC^{low} Treg function.

### Expanded IFNγ⁺IL-10⁺IL-34⁺secreting CDS⁺CD45RC^{low} Tregs display a therapeutic effect in vivo

Finally, the suppressive potential of such expanded CD8⁺CD4SRC^{low} Tregs was assessed using two distinct models of transplantation using NSG mice, a model of allograft rejection of human skin following injection of allogeneic PBMCs **(****Figure 14****)** and a model of xenogeneic GVHD following injection of human PBMCs **(****Figure 15****).**

In these models, syngeneic expanded CD8⁺CD4SRC^{low} Tregs were injected at the same time than PBMCs to assess their potential at inhibiting anti-donor immune responses by measuring percentage of survival, skin histology and weight loss.

Interestingly, in both cases the co-transfer of expanded CD8⁺CD4SRC^{low} Tregs significantly inhibited graft rejection, demonstrating the potential of the CD8⁺CD4SRC^{low} Tregs as a cellular therapy.

### REFERENCES:

Throughout this application, various references describe the state of the art to which this invention pertains.
Feng S. 2008 Long-term management of immunosuppression after pediatric liver transplantation: is minimization or withdrawal desirable or possible or both? Curr Opin Organ Transplant. Oct;13(5):506-12.
Guillonneau, C., Hill, M., Hubert, F.X., Chiffoleau, E., Herve, C., Li, X.L., Heslan, M., Usal, C., Tesson, L., Menoret, S., et al. 2007. CD40Ig treatment results in allograft acceptance mediated by CD8CD45RC T cells, IFN-gamma, and indoleamine 2,3-dioxygenase. J Clin Invest 117:1096-1106.
Li, X.L., Menoret, S., Bezie, S., Caron, L., Chabannes, D., Hill, M., Halary, F., Angin, M., Heslan, M., Usal, C., et al. 2010. Mechanism and localization of CD8 regulatory T cells in a heart transplant model of tolerance. J Immunol 185:823-833.
Picarda, E., Bezie, S., Venturi, V., Echasserieau, K., Merieau, E., Delhumeau, A., Renaudin, K., Brouard, S., Bernardeau, K., Anegon, I., et al. 2014. MHC-derived allopeptide activates TCR-biased CD8+ Tregs and suppresses organ rejection. J Clin Invest 124:2497-2512.
Sadelain M, Brentjens R, Rivière I. 2013 The basic principles of chimeric antigen receptor (CAR) design; Cancer Discov. April ; 3(4): 388-398.
Streuli, M., Hall, L.R., Saga, Y., Schlossman, S.F., and Saito, H. 1987. Differential usage of three exons generates at least five different mRNAs encoding human leukocyte common antigens. J Exp Med 166:1548-1566.

## Claims

1. An isolated population of IFNγ⁺IL-10⁺IL-34⁺ secreting human CD8⁺CD45RC^{low} T regulatory (Treg) cells.

2. The isolated population according to claim **1,** wherein said Treg cells are GITR⁺ and/or Foxp3⁺.

3. The isolated population according to claim **1** or claim **2,** wherein said Treg cells are genetically modified Treg cells comprising a chimeric antigen receptor (CAR) or a chimeric autoantibody receptor (CAAR).

4. A method for detecting and/or isolating IFNγ⁺IL⁻10⁺IL⁻34⁺ secreting human CD8⁺CD45RC^{low} Treg cells from a biological sample containing human peripheral blood mononuclear cells (PBMCs) or lymphocytes, wherein said Treg cells optionally are GITR⁺ and/or Foxp3⁺, comprising the following steps of:
(a) contacting said biological sample containing human PBMCs or lymphocytes with means useful for isolating a population of human CD8⁺CD45RC^{low} Treg cells, optionally with means useful for isolating a population of GITR⁺ and/or Foxp3⁺ CD8⁺CD45RC^{low} Treg cells,
wherein the means useful for isolating the population of human CD8⁺CD45RC^{low} Treg cells are binding partners, preferably antibodies, to suitable cell surfaces molecules or markers selected from CD3, CD8 and CD45RC;
(b) contacting the isolated population of human CD8⁺CD45RC^{low} Treg cells obtained at step (a) with means useful for isolating IFNγ⁺IL⁻10⁺IL⁻34⁺ secreting human CD8⁺CD45RC^{low} Treg cells,
wherein the means useful for isolating IFNγ⁺IL-10⁺IL-34⁺ secreting human CD8⁺CD45RC^{low} Treg cells are a combination of at least two bispecific antibodies selected from the group consisting of a bispecific antibody which binds to IPNγ and to a cell surface molecule specific for T cells selected from CD3, CD8 and CD45RC, a bispecific antibody which binds to IIL-10 and to a cell surface molecule specific for T cells selected from CD3, CD8 and CD45RC, and a bispecific antibody which binds to IL-34 and to a cell surface molecule specific for T cells selected from CD3, CD8 and CD45RC.

5. The method according to claim **4,** wherein the means useful for isolating the population of human CD8⁺CD45RC^{low} Treg cells are a combination of at least three antibodies consisting of a monoclonal anti-human CD3 antibody, a monoclonal anti-human CD8 antibody and a monoclonal anti-human CD45RC antibody, optionally further comprising an anti-human GITR antibody.

6. A method for expanding IFNγ⁺IL-10⁺IL-34⁺ secreting human CD8⁺CD45RC^{low} Treg cells, said Treg cells optionally being GITR⁺ and/or Foxp3⁺, comprising a step of contacting an isolated population of IFNγ⁺IL-10⁺IL-34⁺ secreting human CD8⁺CD45RC^{low} Treg cells with a culture medium suitable for expanding Treg cells.

7. The method according to claim **6,** wherein the isolated population of IFNγ⁺IL-10⁺IL-34⁺ secreting human CD8⁺CD45RC^{low} Treg cells is frozen and subsequently thawed before being contacted with a culture medium suitable for expanding Treg cells.

8. The method according to claim **6** or **7,** wherein said culture medium suitable for expanding Treg cells comprises at least an antigen-specific stimulating agent selected from the group consisting of antigens, cells, MHC polymers and antibodies.

9. The method according to any one of claims **6** to **8,** wherein said culture medium comprises at least one cytokine.

10. The method according to claim **9,** wherein said culture medium comprises an amount of interleukin-2 (IL-2) and/or an amount of interleukin-15 (IL-15).

11. A pharmaceutical composition comprising an isolated population of IFNγ⁺IL-10⁺IL-34⁺ secreting human CD8⁺CD45RC^{low} Treg cells according to any one of claims **1** to **3** and at least one pharmaceutically acceptable carrier.

12. An isolated population of IFNγ⁺IL-10⁺IL-34⁺ secreting human CD8⁺CD45RC^{low} Treg cells according to any one of claims **1 to 3** for use as a medicament.

13. An isolated population of IFNγ⁺IL-10⁺IL-34⁺ secreting human CD8⁺CD45RC^{low} Treg cells according to any one of claims **1** to **3** for use in a method for preventing or treating transplant rejection, GVHD, chronic inflammatory diseases, autoimmune diseases, unwanted immune response against therapeutic proteins or allergies in a patient in need thereof.

14. An *in vitro* method for determining whether a patient is at risk of transplant rejection, GVHD, chronic inflammatory diseases, autoimmune diseases, unwanted immune response against therapeutic proteins or allergies, comprising a step of determining the presence of IFNγ⁺IL-10⁺IL-34⁺ secreting human CD8⁺CD45RC^{low} Treg cells in a biological sample obtained from said patient by a method according to claim **4** or claim **5,** wherein the presence of IFNγ⁺IL-10⁺IL-34⁺ secreting human CD8⁺CD45RC^{low} Treg cells is indicative of a reduced risk of transplant rejection, GVHD, chronic inflammatory diseases, autoimmune diseases, unwanted immune response against therapeutic proteins or allergies.

## Patentansprüche

1. Isolierte Population von IFNγ⁺IL-10⁺IL-34⁺ sezernierenden humanen regulatorischen CD8⁺CD45RC^{low}-T-Zellen (Treg-Zellen).

2. Isolierte Population nach Anspruch **1,** wobei die Treg-Zellen GITR⁺ und/oder Foxp3⁺ sind.

3. Isolierte Population nach Anspruch **1** oder Anspruch **2,** wobei die Treg-Zellen genetisch modifizierte Treg-Zellen sind, umfassend einen chimären Antigenrezeptor (CAR) oder einen chimären Autoantikörperrezeptor (CAAR).

4. Verfahren zum Nachweisen und/oder Isolieren von IFNγ⁺IL-10⁺IL-34⁺ sezernierenden humanen CD8⁺CD45RC^{low}-Treg-Zellen aus einer biologischen Probe, enthaltend humane mononukleäre Zellen des peripheren Bluts (PBMC) oder Lymphozyten, wobei die Treg-Zellen optional GITR⁺ und/oder Foxp3⁺ sind, umfassend die folgenden Schritte:
(a) Inkontaktbringen der biologischen Probe, enthaltend humane (PBMC) oder Lymphozyten mit Mitteln, die zum Isolieren einer Population von humanen CD8⁺CD45RC^{low}-Treg-Zellen nützlich sind, optional mit Mitteln, die zum Isolieren einer Population von GITR⁺- und/oder Foxp3⁺-CD8⁺CD45RC^{low}-Treg-Zellen nützlich sind,
wobei die Mittel, die zum Isolieren der Population von humanen CD8⁺CD45RC^{low}-Treg-Zellen nützlich sind, Bindungspartner, vorzugsweise Antikörper zu geeigneten Zelloberflächenmolekülen oder Markern sind, ausgewählt aus CD3, CD8 und CD45RC;
(b) Inkontaktbringen der isolierten Population von humanen CD8⁺CD45RC^{low}-Treg-Zellen, die bei Schritt (a) erhalten wurden, mit Mitteln, die zum Isolieren von IFNγ⁺IL-10⁺IL-34⁺ sezernierenden humanen CD8⁺CD45RC^{low}-Treg-Zellen nützlich sind,
wobei die Mittel, die zum Isolieren von IFNγ⁺IL-10⁺IL-34⁺ sezernierenden humanen CD8⁺CD45RC^{low}-Treg-Zellen nützlich sind, eine Kombination von mindestens zwei bispezifischen Antikörpern sind, ausgewählt aus der Gruppe bestehend aus einem bispezifischen Antikörper, der an IPNγ und an ein für T-Zellen spezifisches Zelloberflächenmolekül, ausgewählt aus CD3, CD8 und CD45RC, bindet, einem bispezifischen Antikörper, der an IL-10 und an ein für T-Zellen spezifisches Zelloberflächenmolekül, ausgewählt aus CD3, CD8 und CD45RC, bindet, und einem bispezifischen Antikörper, der an IL-34 und an ein für T-Zellen spezifisches Zelloberflächenmolekül, ausgewählt aus CD3, CD8 und CD45RC, bindet.

5. Verfahren nach Anspruch **4,** wobei die Mittel, die zum Isolieren der Population von humanen CD8⁺CD45RC^{low}-Treg-Zellen nützlich sind, eine Kombination von mindestens drei Antikörpern sind, bestehend aus einem monoklonalen Anti-Mensch-CD3-Antikörper, einem monoklonalen Anti-Mensch-CD8-Antikörper und einem monoklonalen Anti-Mensch-CD45RC-Antikörper, optional weiterhin umfassend einen Anti-Mensch-GITR-Antikörper.

6. Verfahren zum Expandieren von IFNγ⁺IL-10⁺IL-34⁺ sezernierenden humanen CD8⁺CD45RC^{low}-Treg-Zellen, wobei die Treg-Zellen optional GITR⁺ und/oder Foxp3⁺ sind, umfassend einen Schritt eines Inkontaktbringens einer isolierten Population von IFNγ⁺IL-10⁺IL-34⁺ sezernierenden humanen CD8⁺CD45RC^{low}-Treg-Zellen mit einem Kulturmedium, das zum Expandieren von Treg-Zellen geeignet ist.

7. Verfahren nach Anspruch 6, wobei die isolierte Population von IFNγ⁺IL-10⁺IL-34⁺ sezernierenden humanen CD8⁺CD45RC^{low}-Treg-Zellen eingefroren und anschließend aufgetaut wird, bevor sie mit einem Kulturmedium, das zum Expandieren von Treg-Zellen geeignet ist, in Kontakt gebracht wird.

8. Verfahren nach Anspruch **6** oder **7,** wobei das Kulturmedium, das zum Expandieren von Treg-Zellen geeignet ist, mindestens ein antigenspezifisches Stimulationsagens umfasst, ausgewählt aus der Gruppe bestehend aus Antigenen, Zellen, MHC-Polymeren und Antikörpern.

9. Verfahren nach einem der Ansprüche **6** bis **8,** wobei das Kulturmedium mindestens ein Zytokin umfasst.

10. Verfahren nach Anspruch **9,** wobei das Kulturmedium eine Menge an Interleukin-2 (IL-2) und/oder eine Menge an Interleukin-15 (IL-15) umfasst.

11. Pharmazeutische Zusammensetzung, umfassend eine isolierte Population von IFNγ⁺IL-10⁺IL-34⁺ sezernierenden humanen CD8⁺CD45RC^{low}-Treg-Zellen nach einem der Ansprüche **1** bis **3** und mindestens einen pharmazeutisch annehmbaren Trägerstoff.

12. Isolierte Population von IFNγ⁺IL-10⁺IL-34⁺ sezernierenden humanen CD8⁺CD45RC^{low}-Treg-Zellen nach einem der Ansprüche **1** bis **3** zur Verwendung als ein Arzneimittel.

13. Isolierte Population von IFNγ⁺IL-10⁺IL-34⁺ sezernierenden humanen CD8⁺CD45RC^{low}-Treg-Zellen nach einem der Ansprüche **1** bis **3** zur Verwendung in einem Verfahren zum Verhindern oder Behandeln von einer Transplantatabstoßung, einer GVHD, chronischen Entzündungskrankheiten, Autoimmunkrankheiten, einer unerwünschten Immunantwort gegen therapeutische Proteine oder Allergien bei einem Patienten, der diesem bedarf.

14. *In-vitro*-Verfahren zum Bestimmen, ob ein Patient ein Risiko von einer Transplantatabstoßung, einer GVHD, chronischen Entzündungskrankheiten, Autoimmunkrankheiten, einer unerwünschten Immunantwort gegen therapeutische Proteine oder Allergien aufweist, umfassend einen Schritt eines Bestimmens des Vorliegens von IFNγ⁺IL-10⁺IL-34⁺ sezernierenden humanen CD8⁺CD45RC^{low}-Treg-Zellen in einer biologischen Probe, die von dem Patienten durch ein Verfahren nach Anspruch **4** oder Anspruch **5** erhalten wurde, wobei das Vorliegen von IFNγ⁺IL-10⁺IL-34⁺ sezernierenden humanen CD8⁺CD45RC^{low}-Treg-Zellen auf ein verringertes Risiko von einer Transplantatabstoßung, einer GVHD, chronischen Entzündungskrankheiten, Autoimmunkrankheiten, einer unerwünschten Immunantwort gegen therapeutische Proteine oder Allergien hinweist.

## Revendications

1. Une population isolée de cellules T CD8⁺CD45RC^{low} régulatrices (Treg) humaines sécrétant IFNγ⁺IL-10⁺IL-34⁺.

2. La population isolée selon la revendication **1,** dans laquelle lesdites cellules Treg sont GITR⁺ et/ou Foxp3⁺.

3. La population isolée selon la revendication **1** ou la revendication **2,** dans laquelle lesdites cellules Treg sont des cellules Treg génétiquement modifiées comprenant un récepteur chimérique d'antigène (CAR) ou un récepteur chimérique d'auto-anticorps (CAAR).

4. Une méthode pour détecter et/ou isoler des cellules Treg CD8⁺CD45RC^{low} humaines sécrétant IFNγ⁺IL-10⁺IL-34⁺ d'un échantillon biologique contenant des cellules sanguines mononuclées périphériques (PBMC) humaines ou des lymphocytes humains, dans laquelle lesdites cellules Treg sont de façon optionnelle GITR⁺ et/ou Foxp3⁺, comprenant les étapes suivantes de :
(a) mettre en contact ledit échantillon biologique contenant des PBMC humaines ou des lymphocytes humains avec des moyens utiles pour isoler une population de cellules Treg CD8⁺CD45RC^{low} humaines, de façon optionnelle avec des moyens utiles pour isoler une population de cellules Treg CD8⁺CD45RC^{low} GITR⁺ et/ou Foxp3⁺,
dans laquelle les moyens utiles pour isoler la population de cellules Treg CD8⁺CD45RC^{low} humaines sont des partenaires de liaison, de préférence des anticorps, de molécules ou de marqueurs de surface cellulaire appropriés sélectionnés parmi CD3, CD8 et CD45RC;
(b) mettre en contact la population isolée de cellules Treg CD8⁺CD45RC^{low} humaines obtenue à l'étape (a) avec des moyens utiles pour isoler des cellules Treg CD8⁺CD45RC^{low} humaines sécrétant IFNγ⁺IL⁻10⁺IL-34⁺,
dans laquelle les moyens utiles pour isoler des cellules Treg CD8⁺CD45RC^{low} humaines sécrétant IFNγ⁺IL⁻10⁺IL⁻34⁺ sont une combinaison d'au moins deux anticorps bispécifiques sélectionnés parmi le groupe consistant en un anticorps bispécifique se liant à IPNγ et à une molécule de surface cellulaire spécifique des cellules T sélectionnée parmi CD3, CD8 et CD45RC, un anticorps bispécifique se liant à IL-10 et à une molécule de surface cellulaire spécifique des cellules T sélectionnée parmi CD3, CD8 et CD45RC, et un anticorps bispécifique se liant à IL-34 et à une molécule de surface cellulaire spécifique des cellules T sélectionnée parmi CD3, CD8 et CD45RC.

5. La méthode selon la revendication **4,** dans laquelle les moyens utiles pour isoler la population de cellules Treg CD8⁺CD45RC^{low} humaines sont une combinaison d'au moins trois anticorps consistant en un anticorps monoclonal anti-CD3 humain, un anticorps monoclonal anti-CD8 humain et un anticorps monoclonal anti-CD45RC humain, de façon optionnelle comprenant en outre un anticorps anti-GITR humain.

6. Une méthode pour expandre des cellules Treg CD8⁺CD45RC^{low} humaines sécrétant IFNγ⁺IL-10⁺IL-34⁺, lesdites cellules Treg étant de façon optionnelle GITR⁺ et/ou Foxp3⁺, comprenant une étape de mise en contact d'une population isolée de cellules Treg CD8⁺CD45RC^{low} humaines sécrétant IFNγ⁺IL-10⁺IL-34⁺ avec un milieu de culture approprié pour l'expansion de cellules Treg.

7. La méthode selon la revendication **6,** dans laquelle la population isolée de cellules Treg CD8⁺CD45RC^{low} humaines sécrétant IFNγ⁺IL-10⁺IL-34⁺ est congelée et est par la suite décongelée avant d'être mise en contact avec un milieu de culture approprié pour l'expansion de cellules Treg.

8. La méthode selon la revendication **6** ou **7,** dans laquelle ledit milieu de culture approprié pour l'expansion des cellules Treg comprend au moins un agent stimulant spécifique d'un antigène sélectionné dans le groupe consistant en des antigènes, des cellules, des polymères de CMH et des anticorps.

9. La méthode selon l'une quelconque des revendications **6** à **8,** dans laquelle ledit milieu de culture comprend au moins une cytokine.

10. La méthode de la revendication **9,** dans laquelle ledit milieu de culture comprend une quantité d'interleurkine-2 (IL-2) et/ou une quantité d'interleukine-15 (IL-15).

11. Une composition pharmaceutique comprenant une population isolée de cellules Treg CD8⁺CD45RC^{low} humaines sécrétant IFNγ⁺IL-10⁺IL-34⁺ selon l'une quelconque des revendications **1** à **3** et au moins un support pharmaceutique acceptable.

12. Une population isolée de cellules Treg CD8⁺CD45RC^{low} humaines sécrétant IFNγ⁺IL-10⁺IL-34⁺ selon l'une quelconque des revendications **1** à **3** pour son utilisation comme médicament.

13. Une population isolée de cellules Treg CD8⁺CD45RC^{low} humaines sécrétant IFNγ⁺IL-10⁺IL-34⁺ selon l'une quelconque des revendications **1** à **3** pour son utilisation dans une méthode pour prévenir ou traiter un rejet de transplant, un GVHD, des maladies inflammatoires chroniques, des maladies auto-immunes, une réponse immunitaire non-désirée contre des protéines thérapeutiques ou des allergies chez un patient en ayant besoin.

14. Une méthode *in vitro* pour déterminer si un patient est à risque d'un rejet de transplant, de GVHD, de maladies inflammatoires chroniques, de maladies auto-immunes, d'une réponse immunitaire non-désirée contre des protéines thérapeutiques ou d'allergies, comprenant une étape de détermination de la présence de cellules Treg CD8⁺CD45RC^{low} humaines sécrétant IFNγ⁺IL-10⁺IL-34⁺ dans un échantillon biologique obtenu dudit patient par une méthode selon la revendication **4** ou la revendication **5,** dans laquelle la présence de cellules Treg CD8⁺CD45RC^{low} humaines sécrétant IFNγ⁺IL-10⁺IL-34⁺ est indicative d'un risque réduit d'un rejet de transplant, de GVHD, de maladies inflammatoires chroniques, de maladies auto-immunes, d'une réponse immunitaire non-désirée contre des protéines thérapeutiques ou d'allergies.
